(19) Europäisches Patentamt
European Patent Office
Office européen des brevets

(11) EP 0 496 369 B1

(12) **EUROPÄISCHE PATENTSCHRIFT**

(45) Veröffentlichungstag und Bekanntmachung des Hinweises auf die Patenterteilung:
**03.04.1996 Patentblatt 1996/14**

(51) Int. Cl.$^6$: **C07D 217/26**

(21) Anmeldenummer: **92100996.5**

(22) Anmeldetag: **22.01.1992**

(54) **Verfahren zur Herstellung der racemischen und optisch aktiven 1,2,3,4-Tetrahydroisochinolin-3-carbonsäure sowie deren Vorprodukte**

Process for the preparation of racemic and optically active-1,2,3,4-tetrahydro isoquinoline 3-carboxylic acid and its precursors

Procédé de préparation de l'acide 1,2,3,4-tetrahydro isoquinoléine-3-carboxylique racemique et optiquement actifs et de ses précurseurs

(84) Benannte Vertragsstaaten:
**AT BE CH DE DK ES FR GB GR IT LI LU NL PT SE**

(30) Priorität: **24.01.1991 DE 4102017**

(43) Veröffentlichungstag der Anmeldung:
**29.07.1992 Patentblatt 1992/31**

(73) Patentinhaber: **HOECHST AKTIENGESELLSCHAFT**
**D-65926 Frankfurt am Main (DE)**

(72) Erfinder:
- **Kammermeier, Bernhard, Dr.**
  **W-6000 Frankfurt am Main (DE)**
- **Lerch, Ulrich, Dr.**
  **W-6238 Hofheim am Taunus (DE)**

(56) Entgegenhaltungen:
**US-A- 4 912 221**
- **BERICHTE DER DEUTSCHEN CHEMISCHEN GESELLSCHAFT Bd. 44, 10. Juli 1911, BERLIN(DE) Seiten 2030 - 2036; A. PICTET UND T. SPENGLER: 'Ueber die Bildung von Isochinolin-derivaten durch Einwirkung von Methylal auf Phenyl-aethylamin, Phenyl-alanin und Tyrosin'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Rank Xerox (UK) Business Services
2.9.16/3.4

## Beschreibung

Die Erfindung betrifft ein Verfahren zur Herstellung der Verbindungen der Formeln I, Ia und Ib, sowie deren Zwischenprodukte.

Verbindungen der Formel Ia oder Ib können anstelle natürlicher Aminosäuren in synthetischen oder halbsynthetischen Peptiden oder peptid-ähnlichen Verbindungen, wie z.B. Bradykinin-Antagonisten oder ACE-Inhibitoren eingesetzt werden und dadurch die metabolische Stabilität und Wirkstärke dieser Verbindungen beträchtlich erhöhen.

Die Verbindungen der Formeln I, Ia und Ib sind bekannt. Gemäß Chem. Ber. 44, 2030 (1911) erhält man die Verbindungen der Formeln I, Ia und Ib durch Cyclisierung racemischen bzw. D- oder L-Phenylalanins mit Formaldehyd und konz. Salzsäure in der Siedehitze. Dieser Syntheseweg birgt allerdings einige gravierende Nachteile:

So racemisiert unter diesen drastischen Versuchsbedingungen ein beträchtlicher Teil des Produkts, wobei die ursprüngliche optische Reinheit verlorengeht (siehe J. Amer. Chem. Soc. 84, 4487 (1962)). Die enantiomerenreinen D- bzw. L-1,2,3,4-Tetrahydroisochinolincarbonsäuren der Formeln Ia bzw. Ib sind dann nur durch sehr aufwendige Reinigungsoperationen zu erhalten (z.B. mehrfaches Umkristallisieren aus der 200fachen Menge Ethanol/Wasser 2:1). Entsprechend liegen die Ausbeuten bei mäßigen 35 - 40 %.

Schwerwiegender ist die Bildung des karzinogenen Bischlormethylethers, der in Mischungen aus Salzsäure und Formaldehyd bei der Cyclisierung von Phenylalanin entsteht. Bischlormethylether wirkt aufgrund seiner alkylierenden Eigenschatten auch am Menschen krebserregend (H.G. Neumann in "Allgemeine und spezielle Pharmakologie und Toxikologie", 4. Aufl., W. Forth, Hrsg., B.I. Wissenschaftsverlag, Mannheim-Wien-Zürich, S. 621 ff (1983)) und kann am Hamster bereits nach einmaliger Exposition von 1 ppm zu bösartigen Tumoren führen (Arch. Environ. Health 30 (2), 61). Daher verbietet sich aus Gründen der Arbeitssicherheit die Anwendung des literaturbekannten Verfahrens.

Der vorliegenden Erfindung liegt daher die Aufgabe zugrunde, Verfahren zur Herstellung von racemischer und enantiomerenreiner D- bzw. L-1,2,3,4-Tetrahydroisochinolin-3-carbonsäure zu finden, welche die beschriebenen Nachteile nicht besitzen.

Diese Aufgabe wird erfindungsgemäß gelöst durch das Verfahren zur Herstellung der Verbindungen der Formein I, Ia und Ib

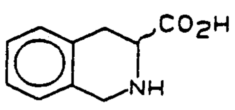
I

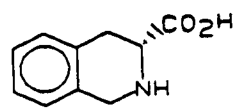
Ia

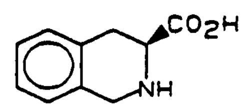
Ib

dadurch gekennzeichnet, daß man

$a_1$) eine Verbindung der Formel IVa oder IVb

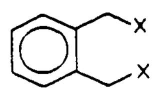

IVa: X=Cl

IVb: X=Br

mit N-Acylamidomalonsäuredialkylestern der Formel $(CO_2R^1)_2CHNHCOR^2$
worin

$R^1$ $(C_1-C_4)$-Alkyl, insbesondere Methyl oder Ethyl und
$R^2$ H, $(C_1-C_4)$-Alkyl oder $(C_6-C_{12})$-Aryl, insbesondere Methyl und Phenyl, bedeuten,

im basischen Milieu zu Verbindungen der Formel II cyclisiert,

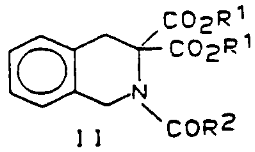

II

worin $R^1$ und $R^2$ wie oben definiert sind,
die so erhaltenen Verbindungen der Formel II durch basische Verseifung und nachfolgende saure Aufarbeitung zu Verbindungen der Formel III

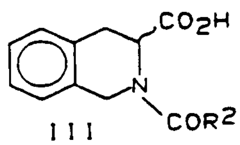

III

decarboxyliert und anschließend im sauren Milieu zu der Verbindung der Formel I umsetzt, oder

$a_2$) die Verbindungen der Formeln IVa bzw. IVb im basischen Milieu zu den Verbindungen der Formel II cyclisiert und diese ohne Isolierung im Eintopfverfahren direkt zur Verbindung der Formel I umsetzt,

gegebenenfalls die racemischen Verbindungen der Formel I

$b_1$) mit (-)Menthol und p-Toluolsulfonsäure zu einem Diastereomerenpaar der Formeln Va bzw. Vb

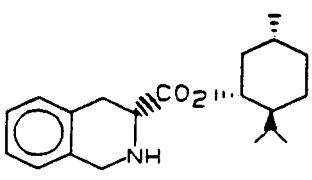

Va

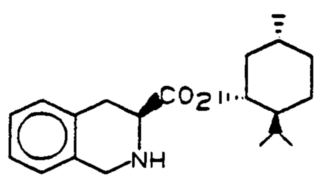

Vb

umsetzt, anschließend die Diastereomeren säulenchromatographisch trennt und zu den Verbindungen der Formeln Ia bzw. Ib basisch verseift oder
$b_2$) die Verbindung der Formel I mittels Benzylalkohol und p-Toluolsulfonsäure zur Verbindung der Formel VI verestert,

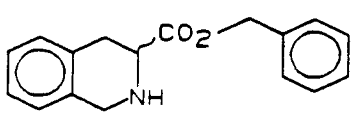

VI

die Verbindung der Formel VI mit D(-)Mandelsäure zu den Verbindungen der Formeln VIIa und VIIb bzw. mit

L(+)Mandelsäure zu den Verbindungen der Formeln VIIIa und VIIIb umsetzt,

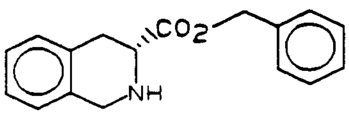

* D (-) Mandelsäure

VIIa

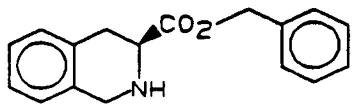

* D (-) Mandelsäure

VIIb

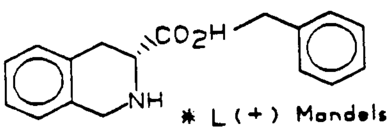

* L (+) Mandelsäure

VIIIa

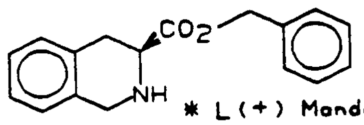

* L (+) Mandelsäure

VIIIb

anschließend die Verbindungen der Formeln VIIa und VIIb bzw. VIIIa und VIIIb durch fraktionierte Kristallisation in einem inerten Lösungsmittel, wie z.B. Essigsäuremethyl-, -ethyl- oder -butylester, Diisopropylether oder MTB, in die optischen Antipoden trennt und die Verbindungen der Formeln Ia und Ib durch basische Verseifung freisetzt, wobei das chirale Hilfsreagenz zurückgewonnen wird.

Wichtige Zwischenprodukte bei diesen Synthesewegen sind

- 1,2,3,4-Tetrahydroisochinolin-N-acyl-3,3-dicarbonsäuredialkylester,
- 1,2,3,4-Tetrahydroisochinolin-N-acyl-3-carbonsäuren,
- (D,L)-1,2,3,4-Tetrahydroisochinolin-3-carbonsäurebenzylester,
- (D)-1,2,3,4-Tetrahydroisochinolin-3-carbonsäurebenzylester-(D)-mandelat
- (L)-1,2,3,4-Tetrahydroisochinolin-3-carbonsäurebenzylester-(D)-mandelat,
- (D)-1,2,3,4-Tetrahydroisochinolin-3-carbonsäurebenzylester-(L)-mandelat,
- (L)-1,2,3,4-Tetrahydroisochinolin-3-carbonsäurebenzylester-(L)-mandelat
- (D)-1,2,3,4-Tetrahydroisochinolin-3-carbonsäure-(-)-menthylester,
- (L)-1,2,3,4-Tetrahydroisochinolin-3-carbonsäure-(-)-menthylester.

Ausgehend von den kommerziell leicht erhältlichen Dihalo-o-xylylenen der Formeln IVa bzw. IVb kann der Dicarbonsäureester der Formel II durch basenkatalysierte Cyclisierung in einem niederen Alkohol, bevorzugt Methanol, mit Acylamidomalonsäuredialkylestern in einfacher Weise dargestellt werden, wobei $R^1$ ($C_1$-$C_4$)-Alkyl, insbesondere Methyl oder Ethyl und $R^2$ H, ($C_1$-$C_4$)-Alkyl oder ($C_6$-$C_{12}$)-Aryl, insbesondere Methyl und Phenyl bedeuten. Als Basen dieser Cyclisierungsreaktion können Alkali- und Erdalkalialkoholate - wie Natriummethanolat, Natriumethanolat, Kalium-tert.-butanolat oder Magnesiumethanolat -, Natriumhydrid oder Alkalihydroxide - wie Natriumhydroxid oder Kaliumhydroxid - verwendet werden. Besonders bevorzugt ist Natriummethanolat. Für größere, insbesondere Technikumansätze wird das Xylylen IVa aus finanziellen und ökologischen Überlegungen bevorzugt.

Der Dicarbonsäureester der Formel II wird mit Alkalihydroxiden, vorzugsweise Natriumhydroxid, bei 20 - 90 °C basisch verseift und mit starken Säuren sauer aufgearbeitet. Man erhält unter Decarboxylierung bei Raumtemperatur die racemische N-Acyl-α-Aminocarbonsäure der Formel III, die nach Behandlung mit einer starken Säure zur racemischen Verbindung der Formel I desacyliert. Unter einer starken Säure wird im allgemeinen HCl, HBr, $H_2SO_4$ und $H_3PO_4$, insbesondere HCl, verstanden.

Besonders bevorzugt ist ein Eintopfverfahren zur Synthese der Verbindung der Formel I, bei welchem das Dichlorxylylen IVa in der oben beschriebenen Weise zum Diester II cyclisiert wird und dieser nach Behandlung mit starker wäßriger Säure, wie z.B. HCl, ohne Isolierung unmittelbar in die Verbindung der Formel I überführt.

Die Reaktionsführung sieht dabei im einzelnen so aus, daß das Lösungsmittel, bevorzugt Methanol, nach der Cyclisierung im Vakuum bei 15 - 60 Torr, bevorzugt 25 Torr, entfernt wird und der feste aus NaCl, Diester der Formel II und kleinen Nebenproduktmengen bestehende Rückstand mit halbkonzentrierter Salzsäure mehrere Stunden am Rückfluß erhitzt wird. Nach beendeter Reaktionszeit wird auf pH 4,5 bis 7 eingestellt, wobei das Produkt der Formel I ausfällt. Die Ausbeuten dieser Eintopfsynthese liegen bei 65 % d.Th. über alle Stufen und sind damit der schrittweisen Synthese mit ca. 70 - 80 % d.Th., ≧ 75 % d.Th. und ca. 70 - 80 % d.Th. über die isolierten Verbindungen II, III und I überlegen. Darüberhinaus gestaltet sich die Reaktionsführung einfacher und der apparative und personelle Aufwand ist wesentlich reduziert.

Die racemisch anfallende α-Aminocarbonsäure der Formel I kann durch eine Veresterung mit (-)Menthol, wie sie für andere neutrale α-Aminosäuren in Chem. Comm 18, 421 (1965) in ähnlicher Weise beschrieben ist, getrennt werden.

Vorzugsweise wird jedoch die α-Aminocarbonsäure der Formel I unter Säurekatalyse, insbesondere mit wasserfreier p-Toluolsulfonsäure in inerten, mit Wasser azeotrop destillierenden Lösungsmitteln wie Benzol, Toluol oder Xylol mit mindestens 1,5 Equivalenten (-)Menthol bei 70 °C bis zur Siedetemperatur des Lösungsmittels zu einer Mischung der diastereomeren (-)Menthylester Va und Vb umgesetzt.
Die Ausbeuten betragen ca. 80 % d.Th. und sind stark von der Reaktionsdauer abhängig, die zwischen 30 und 60 Stunden liegt. Das Rohprodukt, zusammen mit überschüssigem (-)Menthol, wird an der Chromatographiesäule (z.B. Kieselgelsäule: 30 - 70 µm) in die diastereomerenreinen Komponenten Va und Vb getrennt mit wenig polaren Elutionsmitteln, wie z.B. Cyclohexan/Essigester, Essigester/Toluol, Diisopropylether/Toluol oder Hexan/MTB-Ether im Verhältnis 80 : 20 bis 20 : 80, bevorzugt 50 : 50, ggf. auch als Gradienten. Überschüssiges Menthol kann in jedem Fall aus den ersten Fraktionen zurückgewonnen werden.

Die Freisetzung der jeweiligen enantiomerenreinen Aminocarbonsäure Ia bzw. Ib

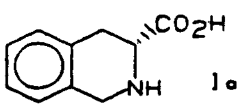 Ia 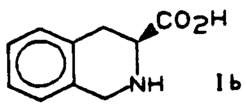 Ib

aus den diastereomerenreinen Menthylestern Va bzw. Vb erfolgt durch Behandlung mit Basen wie Natronlauge oder Kaliumlauge im wäßrigen, oder wäßrig-alkoholischen Milieu , wie z.B. Wasser/$C_1$-$C_3$-Alkylalkohol, im Temperaturbereich von Raumtemperatur bis 90 °C. Die Verbindungen Ia bzw. Ib fallen dabei nach Einstellen eines pH-Wertes zwischen 4,5 und 7,0 in Ausbeuten von ca. 80 % d. Th. und in Enantiomerenreinheiten von ee > 99 % aus. Eine weitere chemische Reinigung etwa durch Umkristallisation ist unnötig.

Ein besonders bevorzugter Weg zur Trennung des Racemats der Formel I in die optischen Antipoden der Formeln Ia bzw. Ib besteht darin, die Verbindung der Formel I in den Benzylester der Formel VI umzusetzen, wie es in ähnlicher Weise für die Verbindungen Ia und Ib in Chem. Pharm. Bull 31 (1), 312 (1983) beschrieben ist.

Die säurekatalysierte Veresterung, vorzugsweise mit wasserfreier p-Toluolsulfonsäure wird wiederum in mit Wasser azeotrop destillierenden Lösungsmitteln wie Toluol oder Xylol mit 3-5 Equivalenten Benzylalkohol bei Rückflußtemperatur durchgeführt. Das entstandene Reaktionswasser wird während der 8-12 stündigen Reaktionsdauer kontinuierlich am Wasserabscheider gesammelt und zeigt den Reaktionsfortgang an. Der racemische Benzylester der Formel VI fällt vorzugsweise als p-Toluolsulfonsäuresalz an und wird aus diesem durch Behandlung mit wäßrigen Basenlösungen, vorzugsweise Alkalicarbonatlösungen in mit Wasser nicht mischbaren organischen Lösungsmitteln, vorzugsweise Essigester, freigesetzt. Nach der Phasentrennung wird die organische Phase mit jeweils equimolaren Mengen D(-) bzw. L(+)Mandelsäure versetzt, wonach die jeweils schwerer löslichen Salzpaare der Formeln VIIa bzw. VIIIb mit Diastereomerenüberschüssen von de > 98 % und chemischen Ausbeuten von ≧ 80 % d. Th. auskristallisieren. Die in der Mutterlauge verbliebenen Reste der jeweils leichter löslichen diastereomeren Salze der Formeln VIIb bzw. VIIIa kristallisieren nach kontinuierlicher Zugabe eines weniger polaren Lösungsmittels, vorzugsweise Diisopropylether oder durch Umsalzen mit dem jeweils anderen Mandelsäureenantiomer, mit Diastereomerenüberschüssen von de = 94 - 99 % und chemischen Ausbeuten von ≧ 70 % d. Th. aus.

Aus den reinen diastereomeren Salzen der Formeln VIIa, VIIb, VIIIa oder VIIIb kann man die Verbindung der Formel Ia bzw. Ib durch basische Verseifung mit 1,0 - 1,1 Equivalenten Alkalihydroxiden, vorzugsweise Natriumhydroxid freisetzen und bei pH 4,5 - 7 und Temperaturen zwischen 0°C und 25°C in Reinheiten von ee > 99 % und chemischen Ausbeuten von ca. 95 % d. Th. ausfällen. Das chirale Hilfsreagens kann aus der Mutterlauge durch Ansäuern und Extraktion zurückgewonnen werden.

Im erfindungsgemäßen Verfahren erfolgt die optische Trennung unter milden Bedingungen, wie z.B. bei 10 °C bis Raumtemperatur, durch fraktionierte Kristallisation unter späterer Rückgewinnung des chiralen Hilfsstoffes (D)- bzw. (L)-Mandelsäure.

Eine optische Spaltung auf der Stufe der racemischen Verbindung der Formel VI ohne optisch aktives Hilfsreagens, etwa durch einfache Umkristallisation ist auch nicht durch Animpfen mit einem optisch reinen Isomer der Verbindung der Formel VI zu erzielen. Die Enantiomerenüberschüsse nach Auskristallisation von 20 - 60 % des Materials liegen im Bereich ee < 5 %.

Eine Spaltung dieser Enantiomeren auf dieser Stufe ist offensichtlich nur zu erreichen, wenn die Enantiomerenüberschüsse des zur Kristallisation eingesetzten Rohmaterials bereits erheblich sind, etwa ee > 80 %, wie in Chem. Pharm. Bull. 31 (1), 312 (1983) beschrieben.

Das erfindungsgemäße Verfahren ist somit dem alten Verfahren der Pictet-Spengler-Cyclokondensation mit Formaldehyd und konzentrierter Salzsäure in jeder Hinsicht überlegen. Es stellt gegenüber dem bisherigen Stand der Technik einen großen Fortschritt dar, weil insbesondere die erwähnten Nachteile der teilweisen Racemisierung der teuren Edukte und der Bildung eines hochtoxischen Nebenproduktes des alten Verfahrens vermieden werden.

Nachfolgende Beispiele sollen die Erfindung näher erläutern.

Beispiel 1

D,L-1,2,3,4-Tetrahydroisochinolin-3-carbonsäure

Methode A: (Verbindung I aus Verbindung IVa)

Es werden bei Zimmertemperatur 5,00 kg α,α'-Dichlor-o-xylylen (Verbindung IVa) und 6,20 kg Acetamidomalonsäurediethylester in 49 l Methanol unter Rühren vorgelegt und während 30 Minuten 5,14 kg Natriummethanolatlösung (30 Gew.-% in Methanol) zudosiert, wobei die Reakionsmischung gleichzeitig bis zum Rückfluß aufgeheizt wird.

Nach beendeter Zugabe wird 10 Minuten unter Rückfluß nachgerührt und anschließend weiter 5,14 kg Natriummethanolatlösung während 2 Stunden unter Rückfluß zudosiert. Anschließend wird 2 Stunden unter Rückfluß nachgerührt, die Mischung schließlich zur Trockne eingeengt und der Rückstand mit 43 l halbkonz. Salzsäure versetzt und erneut 4 Stunden zum Rückfluß gerührt. Die Mischung beginnt dabei kräftig zu schäumen (Decarboxylierung), wobei sich die Gasentwicklung nach 2 Stunden verstärkt.

Nach beendeter Reaktionszeit wird der Ansatz 15 Stunden bei Zimmertemperatur nachgerührt, anschließend auf 0°C gekühlt, mit 25 % Ammoniaklösung unter Kühlung auf pH 6,5 gestellt, eine weitere Stunde bei 0°C nachgerührt und abgesaugt. Der Rückstand wird mit 2 x 2 l Wasser nachgewaschen und im Trockenschrank bei 60°C getrocknet (Karl-Fischer Titration nach 12 Stunden Trocknung ergibt 0,06 % Restwassergehalt).
Ausbeute: 3,27 kg (65 % d. Th. über alle Stufen)
Schmp.: > 300°C
DC: Kieselgel, Merck; Essigester (EE)-MeOH-Eisessig-$H_2O$ 70 : 30 : 5 : 5 $R_f$ : 0,3 (Anfärbung mit Ninhydrin)
$^1$H-NMR ($CF_3CO_2D$, 270 MHz); δ = 3,51 und 3,66 (2 x dd, 2H, $J_{gem}$ = 18 Hz, 9 Hz, 6 Hz; $C\underline{H}_2$-CH); 4,64 - 4,78 (m, 3H, $CH_2$-C$\underline{H}$ und $CH_2$-N); 7,24 - 7,48 (m, 4H, Aromaten-H).

Methode B: (Verbindung I aus Verbindung III)

7,6 g (D,L)-1,2,3,4-Tetrahydroisochinolin-N-acetyl-3-carbonsäure (Verbindung III) werden in 50 ml halbkonz. Salzsäure suspendiert und 4 Stunden am Rückfluß gerührt. Anschließend wird auf 0°C gekühlt, mit 25 % Ammoniaklösung auf pH 6,5 gestellt, eine weitere Stunde bei 0°C nachgerührt und abgesaugt. Der Rückstand wird mit 2 x 10 ml Wasser nachgewaschen, trockengesaugt und im Vakuum-Exsikkator getrocknet.
Ausbeute: 3,6 g (78 % d. Th.).
physikalische Daten: siehe unter Methode A.

Beispiel 2

1,2,3,4-Tetrahydroisochinolin-N-acetyl-3,3-dicarbonsäuredimethylester
(Verbindung II aus Verbindung IVb)

Zu einer gerührten Suspension von 26,4 g α,α'-Dibrom-o-xylilen (Verbindung IVb) und 21,7 g Acetamidomalonsäurediethylester in 175 ml Methanol werden bei Zimmertemperatur 18 ml Natriummethanolatlösung (30 Gew.-% in Methanol) während 10 Minuten zugetropft und anschließend zum Rückfluß erhitzt.

Nach 15 minütigem Rückflußkochen werden weitere 18 ml Natriummethanolatlösung während 2 Stunden zudosiert und nach beendeter Zugabe noch 2 Stunden bei Rückflußtemperatur weitergerührt (ein pH-Wert zeigt dann Neutralität). Zur Aufarbeitung wird das Gemisch zur Trockne eingeengt, der Rückstand zwischen 100 ml Wasser und 150 ml Essigester verteilt und die Phasen getrennt. Die wäßrige Phase wird mit 2 x 75 ml Essigester extrahiert und die vereinigten organischen Phasen mit 1 x 100 ml halbkonz. Kochsalzlösung gewaschen, über $Na_2SO_4$ getrocknet und zur Trockne eingeengt. Der Rückstand wird aus 100 ml Methyl-tert.-butylether (MTB-Ether)/Diisopropylether (1 : 1) umgelöst.
Ausbeute: 21,5 g (75% d. Th.)
Schmp.: 141 - 143°C
DC: Kieselgel, Merck; MTB-Ether
$R_f$: 0,3
$^1$H-NMR ($CDCl_3$, 270 MHz); δ = 2,30 (s, 3H, NCO-$CH_3$); 3,43 (s, 2H, $CH_2$-C); 3,68 (s, 6H, $CO_2CH_3$); 4,68 (s, 2H, $CH_2$-N); 7,13 - 7,27 (m, 4H, Aromaten-H).

Beispiel 3

D,L-1,2,3,4-Tetrahydroisochinolin-N-acetyl-3-carbonsäure
(Verbindung III aus Verbindung II)

Eine Suspension von 10,65 g 1,2,3,4-Tetrahydroisochinolin-N-acetyl-3,3-dicarbonsäuredimethylester (Verbindung II) im Lösungsmittelgemisch von 100 ml Methanol und 20 ml Wasser wird bei Zimmertemperatur portionsweise mit 4,55 g Kaliumhydroxidplätzchen versetzt und anschließend 4 Stunden am Rückfluß erhitzt, wonach eine klare Lösung entsteht.

Die Reaktionslösung wird nach Abkühlen auf Zimmertemperatur zur Trockne eingeengt, der Rückstand mit 100 ml Essigester versetzt und mit 2n-Salzsäure unter kräftigem Rühren auf pH 1 gestellt (Gasentwicklung).

Nach der Phasentrennung wird die wäßrige Phase mit 3 x 50 ml Essigester extrahiert, die vereinigten organischen Phasen über $Na_2SO_4$ getrocknet und zur Trockne eingeengt. Der Rückstand wird mit 10 ml MTB-Ether angeteigt und schließlich trockengesaugt.

Ausbeute: 6,84 g (85 % d. Th.)
Schmp.: 171 - 173°C (Lit: 171 - 172°C)[1]
DC: Kieselgel, Merck; Essigester-MeOH-Eisessig-$H_2O$ = 70 : 30 : 5 : 5
$R_f$: 0,7

$^1$H-NMR ($d_6$-DMSO, 270 MHz); δ = 2,07 und 2,16(2 x s, 3H, $NCOCH_3$, Koaleszenztemp.: 90°C); 3,00 - 3,29 (m, 2H, $C\underline{H_2}$-CH); 4,31, 4,62, 4,74 und 4,75 (2 x 2 x d, 2H, J = 18 Hz, 16 Hz, $CH_2$-N); 4,98 und 5,16 (2 x dd, 1H, J = 6 Hz, 4 Hz, $CH_2$-C$\underline{H}$); 7,13 - 7,25 (m, 4H, Aromaten-H); 12,7 (breit, 1H, $CO_2H$).

Beispiel 4

(D)- und (L)-1,2,3,4-Tetrahydroisochinolin-3-carbonsäure(-)menthylester
(Verbindungen Va und Vb aus Verbindung I)

10 g p-Toluolsulfonsäuremonohydrat werden unter Rühren in 150 ml Toluol suspendiert und 1 Stunde am Wasserabscheider zum Rückfluß erhitzt (im Wasserabscheider verbleiben danach 50 ml Toluol und 1 ml Wasser).

Nun wird die Mischung auf 80 - 90°C abgekühlt, nacheinander mit 6,3 g D,L-1,2,3,4-Tetrahydroisochinolin-3-carbonsäure (Verbindung I) und 8,3 g (-)Menthol versetzt und anschließend weitere 30 Stunden am Wasserabscheider zum Rückfluß erhitzt (nach dieser Zeit sind weitere 1,3 ml Wasser abgeschieden). Die braune Reaktionslösung wird auf Zimmertemperatur abgekühlt und mit 2 x 75 ml 2n Natriumbicarbonatlösung gewaschen. Die vereinigten wäßrigen Phasen werden mit 1 x 50 ml gesättigter Kochsalzlösung gewaschen.

Die organische Phase wird über $Na_2SO_4$ getrocknet und zur Trockne eingeengt, wonach 10,3 g öliges Rohprodukt zurückbleiben. Das Rohprodukt wird an einer Chromatographiesäule über 300 g Kieselgel (30 - 70 µm) mit dem Lösungsmittelgemisch Cyclohexan/Essigester (7 : 3) gereinigt und in die beiden Diastereomeren getrennt.

Ausbeuten: 1,2 g rückgew. (-)Menthol
4,6 g Verbindung Vb (83 % d. Th.); $[\alpha]_D^{20}$ = -117,2° (c = 1, MeOH)
4,2 g Verbindung Va (76 % d. Th.); $[\alpha]_D^{20}$ = -10,5° (c = 0,5, MeOH)

DC: Kieselgel, Merck; Toluol-Essigester 1 : 1

$R_f$: 0,9 ((-)Menthol)
0,6 (Verbindung Vb)
0,5 (Verbindung Va)

$^1$H-NMR ($d_6$-DMSO, 270 MHz, Verbindung Vb): δ = 0,71 (d, 3H, J = 7 Hz; $(CH_2)_2$=CH-C$\underline{H_3}$); 0,84 - 0,92 (m, 6H, CH($\underline{CH_3}$)$_2$); 0,77 - 1,13, 1,25 - 1,55, 1,58 - 1,71 und 1,78 - 1,90 (4 x m, 9H, cyclo-C$\underline{H_2}$-C$\underline{H}$-C$\underline{H_2}$-C$\underline{H_2}$-C$\underline{H}$(C$\underline{H}$)-C(O)); 2,78 und 2,93 (2 x dd, 2H, $J_{gem}$ = 16 Hz, 10 Hz, 5 Hz; C$\underline{H_2}$-CH(N)); 3,64 (dd, 1H, J = 10 Hz, 5Hz; CH-N); 3,90 und 3,95 (2 x d, 2H, J = 16 Hz; $CH_2$-N); 4,66 (dt, 1H, J = 11 Hz, 4 Hz, CH-O); 7,00 - 7,15 (m, 4H, Aromaten-H).

$^1$H-NMR ($d_6$-DMSO, 270 MHz, Verbindung Va): δ = 0,64 (d, 3H, J = 7 Hz; $(CH_2)_2$=CH-C$\underline{H_3}$); 0,82 - 0,90 (m, 6H, CH($\underline{CH_3}$)$_2$); 0,77 - 1,11, 1,22 - 1,55, 1,58 - 1,92 (3 x m, 9H, cyclo-C$\underline{H_2}$-C$\underline{H}$-C$\underline{H_2}$-C$\underline{H_2}$-C$\underline{H}$(C$\underline{H}$)-C(O)-); 2,82 und 2,94 (2 x dd, 2H, $J_{gem}$ = 16 Hz, 10 Hz, 5 Hz; C$\underline{H_2}$-CH(N)); 3,67 (dd, 1H, J = 10 Hz, 5 Hz; CH-N); 3,88 und 3,95 (2 x d, 2H, J = 16 Hz; $CH_2$-N); 4,62 (dt, 1H, J = 11 Hz, 4 Hz; CH-O); 6,98 - 7,13 (m, 4H, Aromaten-H).

[1]Chem. Pharm. Bull. 16(3), 414 (1968)

Beispiel 5

D-1,2,3,4-Tetrahydroisochinolin-3-carbonsäure
(Verbindung Ia aus Verbindung Va)

1,1 g D-1,2,3,4-Tetrahydroisochinolin-3-carbonsäure(-)Menthylester (Verbindung Va) werden bei 0°C vorgelegt, mit einer gleichfalls auf 0°C gekühlten Lösung von 210 mg NaOH in 12 ml Methanol versetzt und 5 Minuten bei dieser Temperatur gerührt. Anschließend wird das Gemisch auf Zimmertemperatur aufgeheizt, weitere 5 Stunden gerührt und schließlich durch Zugabe von 2n-Salzsäure auf pH 7 gebracht.

Zur vollständigen Kristallisation wird das Gemisch ohne Rühren 12 Stunden bei Zimmertemperatur belassen, anschließend abgesaugt, mit 5 ml Wasser nachgewaschen und im Vakuum bei Zimmertemperatur getrocknet.
Ausbeute: 515 mg (≙ 83 % d. Th.) $[\alpha]_D^{20}$ = 130° (c = 1; 0, 1n HCl); ee > 99% (GC, Cyclodextrinsäule)
Schmp.: 321 - 323°C
DC: Kieselgel, Merck; Essigester-MeOH-Eisessig-$H_2O$ = 70 : 30 : 5 : 5
$R_f$: 0,3 (Anfärbung mit Ninhydrin)
$^1$H-NMR analog Verbindung I

Beispiel 6

D,L-1,2,3,4-Tetrahydroisochinolin-3-carbonsäurebenzylester
(Verbindung VI aus Verbindung I)

190,2 g p-Toluolsulfonsäuremonohydrat werden unter Rühren in 3 l Toluol suspendiert und 1 Stunde am Wasserabscheider zum Rückfluß erhitzt. Nun wird die Temperatur auf 80 - 90°C reduziert, 177,2 g D,L-1,2,3,4-Tetrahydroisochinolin-3-carbonsäure eingetragen und eine weitere Stunde am Rückfluß erhitzt (im Wasserabscheider verbleiben dann 80 ml Toluol und 22 ml Wasser).

Zur Reaktionslösung werden nun während 30 Minuten 300 ml Benzylalkohol getropft und nach beendeter Zugabe weitere 10 Stunden am Wasserabscheider zum Rückfluß erhitzt. Die klare, braune Reaktionslösung wird zur Trockne eingeengt und anschließend mit 2 l Essigester und 1 l Diisopropylether verrührt, wonach das Produkt spontan auszukristallisieren beginnt. Das Gemisch wird unter Rühren mit weiteren 2 l Diisopropylether versetzt, 2 Stunden bei 0°C gerührt und abgesaugt. Der Rückstand wird mit 100 ml Diisopropylether nachgewaschen und trockengesaugt. Der Rückstand wird im Vakuum bei Zimmertemperatur getrocknet.
Ausbeute: 346,5 g (79 % d. Th. an VI·Toluolsulfonsäure)
Schmp.: 128 - 130°C
DC: Kieselgel, Merck; Essigester-MeOH-Eisessig-$H_2O$ = 70 : 30 : 5 : 5
$R_f$: 0,8
$^1$H-NMR ($d_6$-DMSO, 270 MHz); δ = 2,29 (s, 3H, Tosyl-$CH_3$); 3,15 und 3,36 (2 x dd, 2H, $J_{gem}$ = 18 Hz, 12 Hz, 5 Hz; C<u>H</u>$_2$-CH); 4,34 und 4,40 (2 x d, 2H, J = 16 Hz; $CH_2$-N); 4,67 (dd, 1H, J = 11 Hz, 4 Hz; CH-N); 5,32 (s, 2H, C<u>H</u>$_2$-Phenyl); 7,11 und 7,47 (2 x d, 4H, J = 8 Hz; Tosyl-H); 7,23 - 7,31 (m, 4H, Phenylen-H); 7,36 - 7,46 (m, 5H, Phenyl-H); 9,70 (breit, 2H, $NH_2^+$).

Verbindung VI wird durch Behandlung mit gesättigter Natriumbicarbonatlösung und Essigesterextraktion als farbloses Öl in quantitativer Ausbeute aus seinem Tosylataddukt freigesetzt:
$^1$H-NMR ($d_6$-DMSO,, 270 MHz); δ = 2,86 und 2,98 (2 x dd, 2H, $J_{gem}$ = 16 Hz, 10 Hz, 5 Hz; C<u>H</u>$_2$-CH); 3,77 (dd, 1H, J = 12 Hz, 4 Hz; CH-N); 3,89 und 3,96 (2 x d, 2H, J = 16 Hz, $CH_2$-N); 5,17 (s, 2H, C<u>H</u>$_2$-Phenyl); 7,00 - 7,15 (m, 4H, Phenylen-H); 7,31 - 7,42 (m, 5H, Phenyl-H).

Beispiel 7

(D)-1,2,3,4-Tetrahydroisochinolin-3-carbonsäurebenzylester-(D)-mandelat,
(L)-1,2,3,4-Tetrahydroisochinolin-3-carbonsäurebenzylester-(D)-mandelat
(Verbindungen VIIa und VIIb aus Verbindung VI)

Unter Rühren und ständiger pH-Kontrolle wird eine Suspension von 109,9 g (D,L)-1,2,3,4-Tetrahydroisochinolin-3-carbonsäure-benzylestertosylat in 500 ml Essigester bei Zimmertemperatur mit gesättigter Natriumbicarbonatlösung zwischen pH 7,0 und 7,5 gestellt und anschließend die Phasen getrennt.

Die Wasserphase wird mit 100 ml Essigester extrahiert und die vereinigten organischen Phasen mit 100 ml Wasser gewaschen. Die Essigesterphase wird über $Na_2SO_4$ getrocknet, filtriert und der Trockenmittelrückstand mit 2 x 50 ml Essigester nachgewaschen. Das Filtrat wird portionsweise mit ingesamt 38,04 g D(-)Mandelsäure versetzt und 12 Stunden bei Zimmertemperatur sowie 1 Stunde bei 0°C gerührt. Nun wird der ausgefallene, farblose Kristallbrei abgesaugt,

mit 50 ml Essigester und 50 ml Diisopropylether nachgewaschen und im Vakuumexsikkator zur Gewichtskonstanz getrocknet.

Verbliebenes (L,D)-diastereomeres Salz (Verbindung VIIb) kristallisiert nach Einengen der Mutterlauge auf ca. 30 % des ursprünglichen Volumens und 10 stündigem Rühren bei Zimmertemperatur aus. Die Mischung wird mit 150 ml Diisopropylether versetzt, kurz gerührt und der Kristallbrei ab- und trockengesaugt.

Ausbeute: 46,6 g Verbindung VIIa (89 % d. Th. an (D,D)-Diastereomer) $[\alpha]_D^{20}$ = +11,7° (c = 1, MeOH); de > 98 % (HPLC nach Derivatisierung)

Schmp.: 98 - 100°C

Ausbeute: 42,3 g Verbindung VIIb (81 % d. Th. an (D,L)-Diastereomer) $[\alpha]_D^{20}$ = -99,6° (c = 0,5 MeOH); de > 96 % (HPLC nach Derivatisierung)

Schmp.: 84 - 87°C

$^1$H-NMR ($d_6$-DMSO, 270 MHz, Verbindung VIIa); δ = 2,88 und 3,02 (2 x dd, 2H, $J_{gem}$ = 16 Hz, 10 Hz, 5 Hz; C$\underline{H}_2$-CH); 3,83 (dd, 1H, J = 11 Hz, 4 Hz; CH-N); 3,94 und 4,00 (2 x d, 2H, J = 16 Hz; $CH_2$-N); 4,98 (s, 1H, Phenyl-C$\underline{H}$-O); 5,18 (s, 2H, Phenyl-$CH_2$-O); 7,02 - 7,15 (m, 4H, Phenylen-H); 7,20 -7,47 (m, 10H, Phenyl-H).

$^1$H-NMR (Verbindung VIIb) identisch zu Verbindung VIIa.

Beispiel 8

(D)-1,2,3,4-Tetrahydroisochinolin-3-carbonsäurebenzylester-(L)-mandelat
(L)-1,2,3,4-Tetrahydroisochinolin-3-carbonsäurebenzylester-(L)-mandelat

Methode A
(Verbindungen VIIIa und VIIIb aus Verbindung VI)

11 g (D,L)-1,2,3,4-Tetrahydroisochinolin-3-carbonsäurebenzylestertosylat werden in 50 ml Essigester suspendiert und bei Zimmertemperatur unter Rühren mit gesättigter Natriumbicarbonatlösung zwischen pH 7,0 und 7,5 gestellt und anschließend die Phasen getrennt. Die Wasserphase wird mit 10 ml Essigester extrahiert und die vereinigten organischen Phasen mit 10 ml Wasser gewaschen. Die organische Phase wird über $Na_2SO_4$ getrocknet, filtriert und der Trokkenmittelrückstand mit 10 ml Essigester nachgewaschen. Das Filtrat wird portionsweise mit 3,8 g L(+)Mandelsäure versetzt und 12 Stunden bei Zimmertemperatur gerührt. Anschließend wird der Niederschlag abgesaugt, mit 20 ml Diisopropylether nachgewaschen und zur Gewichtskonstanz getrocknet. Die erhaltene Mutterlauge wird zur Kristallisation des darin verbliebenen (D,L)-diastereomeren Salzes (Verbindung VIIIa) auf ca. 30 % des ursprünglichen Volumens eingeengt, weitere 3 - 4 Stunden bei 0°C gerührt und der entstandene dicke Kristallbrei mit 10 ml Essigester und 20 ml Diisopropylether versetzt. Nach anschließendem 15 minütigen Rühren bei Zimmertemperatur wird ab- und trockengesaugt.

Ausbeute: 4,30 g Verbindung VIIIb (82 % d. Th. an (L,L)-Diastereomer) $[\alpha]_D^{20}$ = +12,7° (c = 1, MeOH); de > 98 % (HPLC nach Derivatisierung)

Schmp.: 99 - 101°C

Ausbeute: 3,80 g Verbindung VIIIa (72 % d. Th. an (D,L)-Diastereomer) $[\alpha]_D^{20}$ = -99,8° (c = 1 MeOH); de > 98 % (HPLC nach Derivatisierung)

Schmp.: 85 - 87°C

$^1$H-NMR ($d_6$-DMSO, 270 MHz, Verbindungen VIIIa und VIIIb) ist identisch zum entsprechenden Spektrum der Verbindung VIIa.

Methode B
(Verbindung VIIIa und VIIIb aus Verbindung VI)

110 g (D,L)-1,2,3,4-Tetrahydroisochinolin-3-carbonsäurebenzylestertosylat werden in 350 ml Essigester suspendiert, bei Zimmertemperatur unter Rühren mit 150 ml gesättigter Natriumbicarbonatlösung und 50 ml Wasser versetzt und nach 10 Minuten die Phasen getrennt. Die organische Phase wird erneut mit 100 ml halbgesättigter Natriumbicarbonatlösung gewaschen, die Phasen getrennt und die vereinigten Wasserphasen mit 150 ml Essigester extrahiert. Die vereinigten Essigesterphasen werden nach dem Waschen mit 100 ml gesättigter Kochsalzlösung über $Na_2SO_4$ getrocknet, filtriert und unter Rühren mit 38 g L(+)Mandelsäure versetzt.

Etwa 5 Minuten nach der Zugabe beginnt die Kristallisation, die durch 10 minütiges Rühren bei Zimmertemperatur und anschließendes einstündiges Rühren bei 0°C vervollständigt wird. Der Kristallbrei wird abgesaugt, mit wenig Essigester und 100 ml Diisopropylether nachgewaschen und Verbindung VIIIa zur Gewichtskonstanz getrocknet. Die verbliebene Mutterlauge wird analog zum oben beschriebenen Vorgehen mit Natriumbicarbonatlösung gewaschen, über $Na_2SO_4$ getrocknet, filtriert und unter Rühren mit 18 g D(-)Mandelsäure versetzt. Die Lösung wird 5 Minuten bei Zimmertemperatur und 1 Stunde bei 0°C gerührt, anschließend vom Kristallbrei abgesaugt, mit wenig Essigester und 50

ml Diisopropylether nachgewaschen und trockengesaugt.
Ausbeute: 50,0 g Verbindung VIIIb (95 % d. Th. an (L,L)-Diastereomer) $[\alpha]_D^{20}$ = - 13,3° (c = 1, MeOH); de > 98 % (HPLC nach Derivatisierung)
Ausbeute: 44,6 g Verbindung VIIIa (85 % d. Th. an (D,D)-Diastereomer) $[\alpha]_D^{20}$ = +14,3° (c = 1 MeOH); de > 99 % (HPLC nach Derivatisierung)

Beispiel 9a

(D)-1,2,3,4-Tetrahydroisochinolin-3-carbonsäure
(Verbindung Ia aus Verbindung VIIa)

Zu einer bei Zimmertemperatur gerührten Suspension von 40 g (D)-1,2,3,4-Tetrahydroisochinolin-3-carbonsäurebenzylester-(D)mandelat in 200 ml Wasser wird während 10 Minuten eine Lösung von 8,4 g NaOH in 200 ml Wasser zudosiert und die Reaktionsmischung dann 9 Stunden weitergerührt.

Die klare Lösung wird mit 2n-Salzsäure auf pH 7 gestellt, wobei das Produkt ausfällt. Die Mischung wird 30 Minuten nachgerührt, zur vollständigen Kristallisation 12 Stunden ohne Rühren bei Raumtemperatur belassen und der Kristallbrei dann abgesaugt.

Der Rückstand wird mit 2 x 15 ml Essigester angeteigt, trockengesaugt und im Vakuum bei Zimmertemperatur getrocknet. Die zweiphasige Mutterlauge wird mit konz. Salzsäure auf pH 1 gestellt und mit 2 x 150 ml Essigester extrahiert. Die vereinigten organischen Phasen werden mit 1 x 50 ml gesättigter Kochsalzlösung gewaschen, von der wäßrigen Phase getrennt, über $Na_2SO_4$ getrocknet und zur Trockne eingeengt. Der Rückstand wird mit 10 ml Toluol angeteigt, trockengesaugt und aus 20 ml Wasser umkristallisiert.
Ausbeute (Verbindung Ia): 15,0 g (89 % d. Th.);
Schmp.: 321 - 325°C; $[\alpha]_D^{20}$ = 130° (c = 1, 0,1 n HCl); ee > 99 % (GC, Cyclodextrinsäule)
Ausbeute (D(-)Mandelsäure): 8,9 g (62 %) $[\alpha]_D^{20}$ = -155,7° (c = 5; $H_2O$) [Lit.: -155 ± 5°]

Beispiel 9b

L-1,2,3,4-Tetrahydroisochinolin-3-carbonsäure
(Verbindung Ib aus Verbindung VIIb)

2,7 g (L)-1,2,3,4-Tetrahydroisochinolin-3-carbonsäurebenzylester-(L)-mandelat werden in 50 ml Essigester suspendiert und mit 2 x 75 ml gesättigter Natriumbicarbonatlösung gewaschen. Die vereinigten wäßrigen Phasen[2] werden mit 30 ml Essigester extrahiert und die vereinigten organischen Phasen mit 50 ml gesättigter Kochsalzlösung[2] gewaschen. Die Essigesterphase wird über $Na_2SO_4$ getrocknet, filtriert und am Rotationsverdampfer zur Trockne eingeengt.

Der ölige Rückstand wird mit einer Lösung von 310 mg NaOH in 25 ml Wasser versetzt und 15 Stunden bei Zimmertemperatur gerührt.

Nach beendeter Reaktionszeit wird die klare Lösung mit 2n-Salzsäure auf pH 4,5 gestellt, 1 Stunde nachgerührt und der farblose Niederschlag abgesaugt und zur Gewichtskonstanz getrocknet.

Ausbeute (Verbindung Ib):
920 mg (80 % d. Th.) $[\alpha]_D^{20}$ = -138° (c = 1, 0, 1n HCl)
Ausbeute (L(+)Mandelsäure):
840 mg (86 % d. Th.) $[\alpha]_D^{20}$ = +154,8° (c = 5, $H_2O$)

Die Verbindungen der Formeln Ia und Ib wurden analog Beispiel 9a und b aus den Verbindungen der Formeln VIIIa bzw. VIIb erhalten.

[2]Zur Mandelsäurerückgewinnung werden diese wäßrigen Phasen vereinigt, mit konz. Salzsäure auf pH = 0-1 gestellt und mit 3 x 20 ml Essigester extrahiert. Die vereinigten organischen Phasen werden mit 10 ml gesättigter Kochsalzlösung gewaschen, von der wäßrigen Phase getrennt, über $Na_2SO_4$ getrocknet und zur Trockne eingeengt. Der farblose Rückstand wird aus Wasser umkristallisiert.

Das folgende Reaktionsschema erläutert die beschriebene Reaktionsfolge:

Schema 1

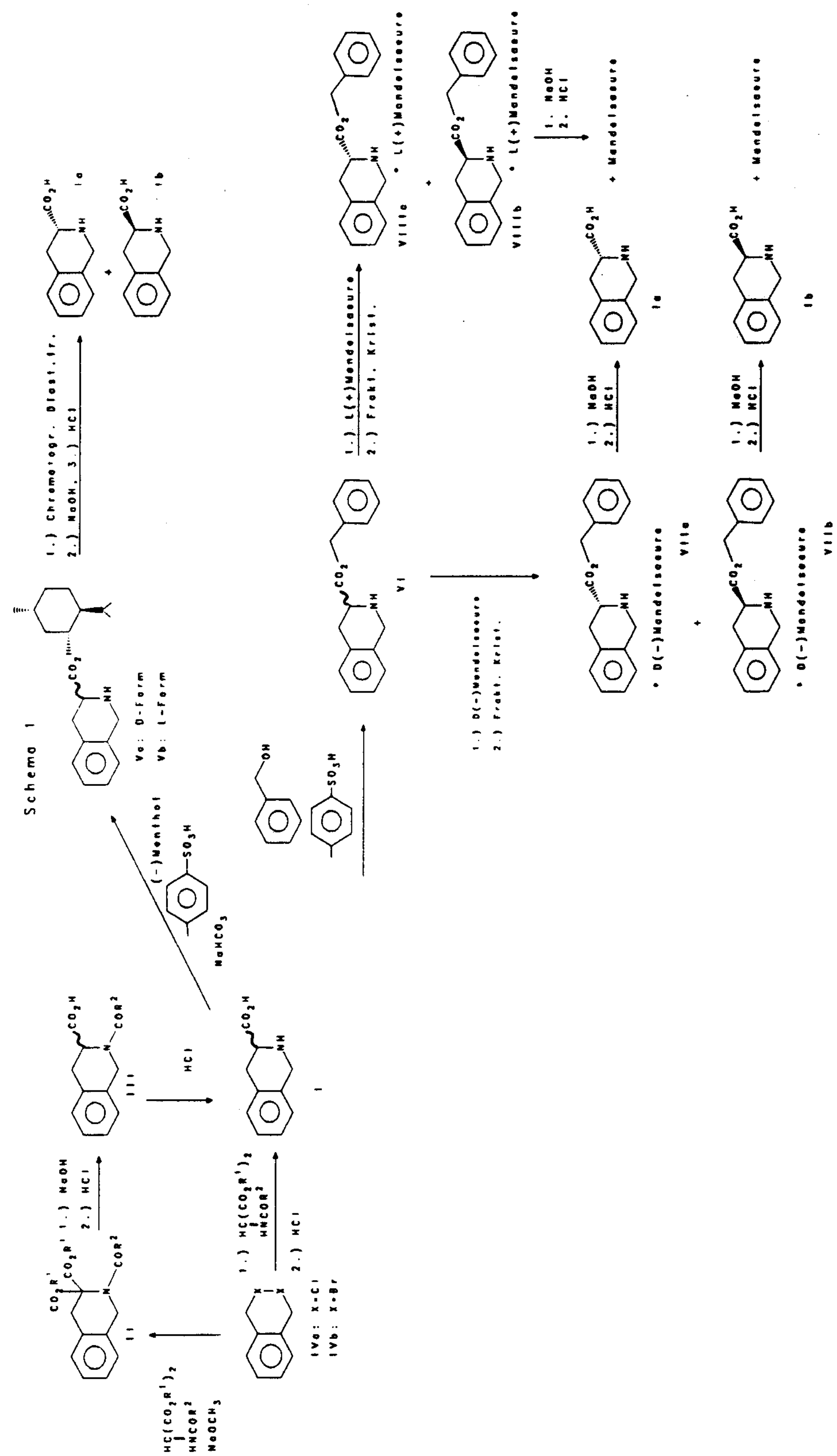

## Patentansprüche

### Patentansprüche für folgende Vertragsstaaten : AT, BE, CH, DE, DK, FR, GB, GR, IT, LI, LU, NL, PT, SE

1. Verfahren zur Herstellung eine Verbindung der Formel I, Ia oder Ib

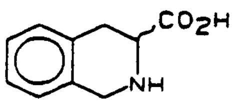

I

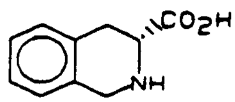

Ia

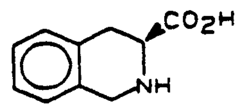

Ib

dadurch gekennzeichnet, daß man

a₁) eine Verbindung der Formel IVa oder IVb

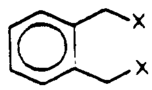

IVa: X=Cl

IVb: X=Br

mit N-Acylamidomalonsäuredialkylestern der Formel $(CO_2R^1)_2CHNHCOR^2$ worin

$R^1$ $(C_1\text{-}C_4)$-Alkyl und
$R^2$ H, $(C_1\text{-}C_4)$-Alkyl oder $(C_6\text{-}C_{12})$-Aryl bedeuten,

im basischen Milieu zu Verbindungen der Formel II cyclisiert,

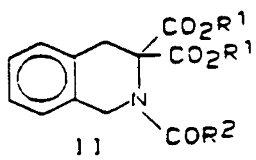

II

worin $R^1$ und $R^2$ wie oben definiert sind,
die so erhaltenen Verbindungen der Formel II durch basische Verseifung und nachfolgende saure Aufarbeitung zu Verbindungen der Formel III

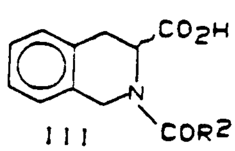

III

decarboxyliert und anschließend im sauren Milieu zu der Verbindung der Formel I umsetzt, oder
$a_2$) die Verbindungen der Formeln IVa bzw. IVb im basischen Milieu zu den. Verbindungen der Formel II cyclisiert und diese ohne Isolierung im Eintopfverfahren direkt zur Verbindung der Formel I umsetzt,

gegebenenfalls die racemischen Verbindungen der Formel I

$b_1$) mit (-)Menthol und p-Toluolsulfonsäure zu einem Diastereomerenpaar der Formeln Va bzw. Vb

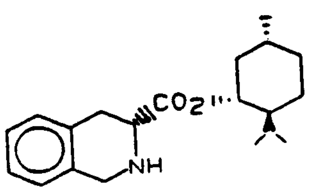

Va

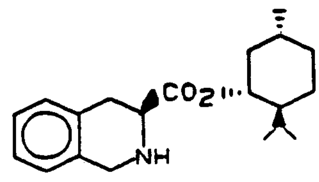

Vb

umsetzt, anschließend die Diastereomeren säulenchromatographisch trennt und zu den Verbindungen der Formeln Ia bzw. Ib basisch verseift oder

$b_2$) die Verbindung der Formel I mittels Benzylalkohol und p-Toluolsulfonsäure zur Verbindung der Formel VI verestert,

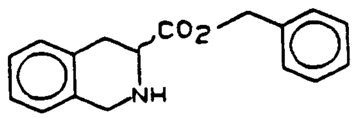

VI

die Verbindung der Formel VI mit D(-)Mandelsäure zu den Verbindungen der Formeln VIIa und VIIb bzw. mit L(+)Mandelsäure zu den Verbindungen der Formeln VIIIa und VIIIb umsetzt,

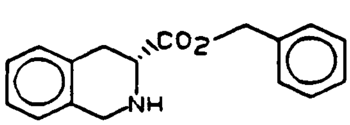

* D (-) Mandelsäure

VIIa

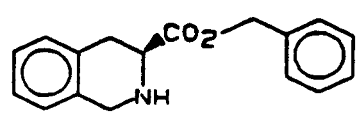

* D (-) Mandelsäure

VIIb

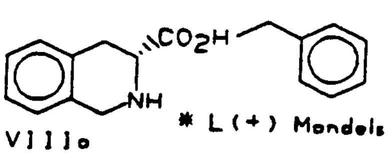

VIIIa

* L (+) Mandelsäure

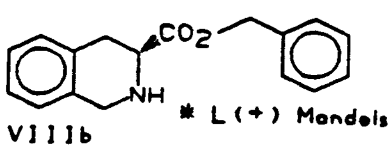

VIIIb

* L (+) Mandelsäure

anschließend die Verbindungen der Formeln VIIa und VIIb bzw. VIIIa und VIIIb durch fraktionierte Kristallisation in einem inerten Lösungsmittel in die optischen Antipoden trennt und die Verbindungen der Formeln Ia und Ib durch basische Verseifung freisetzt, wobei das chirale Hilfsreagenz zurückgewonnen wird.

2. Verfahren zur Herstellung einer Verbindung der Formel I, Ia oder Ib gemäß Anspruch 1, dadurch gekennzeichnet, daß man die eine Verbindung der Formel IVa oder IVb im Eintopfverfahren zur Verbindung der Formel I umsetzt und gegebenenfalls die so erhaltene Verbindung der Formel I mittels Benzylalkohol und p-Toluolsulfonsäure zur Verbindung der Formel VI verestert, die Verbindung der Formel VI mit D(-)Mandelsäure zu den Verbindungen der Formeln VIIa und VIIb bzw. mit L(+)Mandelsäure zu den Verbindungen der Formeln VIIIa und VIIIb umsetzt, anschließend die Verbindungen der Formeln VIIa und VIIb bzw. VIIIa und VIIIb durch fraktionierte Kristallisation in einem inerten Lösungsmittel in die optischen Antipoden trennt und die Verbindungen der Formeln Ia und Ib durch basische Verseifung freisetzt, wobei das chirale Hilfsreagenz zurückgewonnen wird.

3. Verfahren zur Herstellung einer Verbindung der Formel Ia oder Ib gemäß Anspruch 1, dadurch gekennzeichnet, daß man die racemische Verbindung der Formel I mit (-)Menthol und p-Toluolsulfonsäure zu einem Diastereomerenpaar der Formeln Va bzw. Vb umsetzt, anschließend die Diastereomeren säulenchromatographisch trennt und zu den Verbindungen der Formeln Ia bzw. Ib basisch verseift oder die Verbindung der Formel I mittels Benzylalkohol

und p-Toluolsulfonsäure zur Verbindung der Formel VI verestert, die Verbindung der Formel VI mit D(-)Mandelsäure zu den Verbindungen der Formeln VIIa und VIIb bzw. mit L(+)Mandelsäure zu den Verbindungen der Formeln VIIIa und VIIIb umsetzt, anschließend die Verbindungen der Formeln VIIa und VIIb bzw. VIIIa und VIIIb durch fraktionierte Kristallisation in einem inerten Lösungsmittel in die optischen Antipoden trennt und die Verbindungen der Formeln Ia und Ib durch basische Verseifung freisetzt, wobei das chirale Hilfsreagenz zurückgewonnen wird.

**4.** 1,2,3,4-Tetrahydroisochinolin-N-acyl-3,3-dicarbonsäuredialkylester,
1,2,3,4-Tetrahydroisochinolin-N-acyl-3,3-dicarbonsäuredimethylester,
(D)-1,2,3,4-Tetrahydroisochinolin-3-carbonsäurebenzylester-(D)-mandelat
(L)-1,2,3,4-Tetrahydroisochinolin-3-carbonsäurebenzylester-(D)-mandelat,
(D)-1,2,3,4-Tetrahydroisochinolin-3-carbonsäurebenzylester-(L)-mandelat,
(L)-1,2,3,4-Tetrahydroisochinolin-3-carbonsäurebenzylester-(L)-mandelat
(D,L)-1,2,3,4-Tetrahydroisochinolin-3-carbonsäure-(-)-menthylester.

**Patentansprüche für folgenden Vertragsstaat : ES**

**1.** Verfahren zur Herstellung einer Verbindung der Formel I, Ia oder Ib

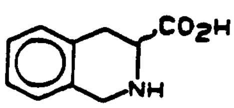
I

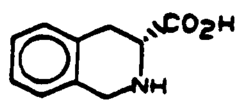
Ia

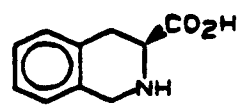
Ib

dadurch gekennzeichnet, daß man

$a_1$) eine Verbindung der Formel IVa oder IVb

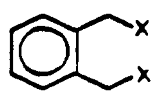

IVa: X=Cl

IVb: X=Br

mit N-Acylamidomalonsäuredialkylestern der Formel $(CO_2R^1)_2CHNHCOR^2$
worin

$R^1$ $(C_1\text{-}C_4)$-Alkyl und
$R^2$ H, $(C_1\text{-}C_4)$-Alkyl oder $(C_6\text{-}C_{12})$-Aryl bedeuten,

im basischen Milieu zu Verbindungen der Formel II cyclisiert,

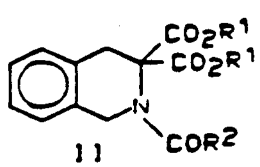
II

worin $R^1$ und $R^2$ wie oben definiert sind,
die so erhaltenen Verbindungen der Formel II durch basische Verseifung und nachfolgende saure Aufarbeitung

zu Verbindungen der Formel III

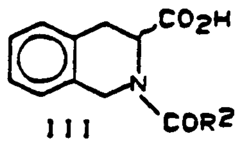

III

decarboxyliert und anschließend im sauren Milieu zu der Verbindung der Formel I umsetzt, oder

$a_2$) die Verbindungen der Formeln IVa bzw. IVb im basischen Milieu zu den Verbindungen der Formel II cyclisiert und diese ohne Isolierung im Eintopfverfahren direkt zur Verbindung der Formel I umsetzt,

gegebenenfalls die racemischen Verbindungen der Formel I

$b_1$) mit (-)Menthol und p-Toluolsulfonsäure zu einem Diastereomerenpaar der Formeln Va bzw. Vb

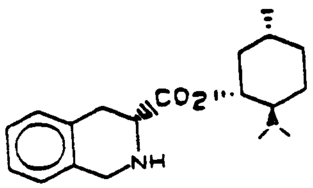

Va

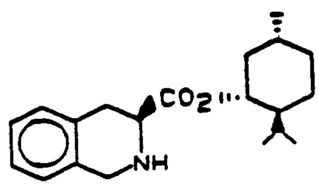

Vb

umsetzt, anschließend die Diastereomeren säulenchromatographisch trennt und zu den Verbindungen der Formeln Ia bzw. Ib basisch verseift oder

$b_2$) die Verbindung der Formel I mittels Benzylalkohol und p-Toluolsulfonsäure zur Verbindung der Formel VI verestert,

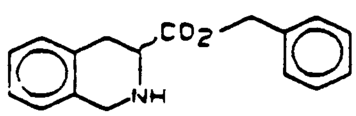

VI

die Verbindung der Formel VI mit D(-)Mandelsäure zu den Verbindungen der Formeln VIIa und VIIb bzw. mit L(+)Mandelsäure zu den Verbindungen der Formeln VIIIa und VIIIb umsetzt,

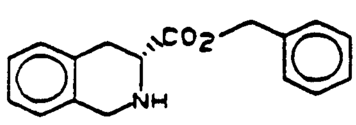

* D (-) Mandelsaure

VIIa

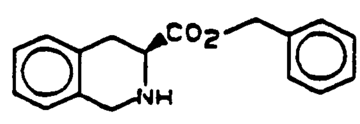

* D (-) Mandelsaure

VIIb

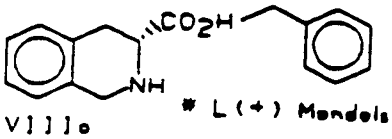

VIIIa * L (+) Mandelsaure

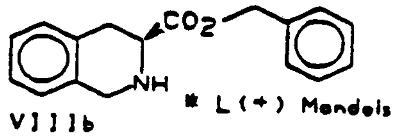

VIIIb * L (+) Mandelsaure

anschließend die Verbindungen der Formeln VIIa und VIIb bzw. VIIIa und VIIIb durch fraktionierte Kristallisation

in einem inerten Lösungsmittel in die optischen Antipoden trennt und die Verbindungen der Formeln Ia und Ib durch basische Verseifung freisetzt, wobei das chirale Hilfsreagenz zurückgewonnen wird.

2. Verfahren zur Herstellung einer Verbindung der Formel I, Ia oder Ib gemäß Anspruch 1, dadurch gekennzeichnet, daß man die eine Verbindung der Formel IVa oder IVb im Eintopfverfahren zur Verbindung der Formel I umsetzt und gegebenenfalls die so erhaltene Verbindung der Formel I mittels Benzylalkohol und p-Toluolsulfonsäure zur Verbindung der Formel VI verestert, die Verbindung der Formel VI mit D(-)Mandelsäure zu den Verbindungen der Formeln VIIa und VIIb bzw. mit L(+)Mandelsäure zu den Verbindungen der Formeln VIIIa und VIIIb umsetzt, anschließend die Verbindungen der Formeln VIIa und VIIb bzw. VIIIa und VIIIb durch fraktionierte Kristallisation in einem inerten Lösungsmittel in die optischen Antipoden trennt und die Verbindungen der Formeln Ia und Ib durch basische Verseifung freisetzt, wobei das chirale Hilfsreagenz zurückgewonnen wird.

3. Verfahren zur Herstellung einer Verbindung der Formel Ia oder Ib gemäß Anspruch 1, dadurch gekennzeichnet, daß man die racemische Verbindung der Formel I mit (-)Menthol und p-Toluolsulfonsäure zu einem Diastereomerenpaar der Formeln Va bzw. Vb umsetzt, anschließend die Diastereomeren säulenchromatographisch trennt und zu den Verbindungen der Formeln Ia bzw. Ib basisch verseift oder die Verbindung der Formel I mittels Benzylalkohol und p-Toluolsulfonsäure zur Verbindung der Formel VI verestert, die Verbindung der Formel VI mit D(-)Mandelsäure zu den Verbindungen der Formeln VIIa und VIIb bzw. mit L(+)Mandelsäure zu den Verbindungen der Formeln VIIIa und VIIIb umsetzt, anschließend die Verbindungen der Formeln VIIa und VIIb bzw. VIIIa und VIIIb durch fraktionierte Kristallisation in einem inerten Lösungsmittel in die optischen Antipoden trennt und die Verbindungen der Formeln Ia und Ib durch basische Verseifung freisetzt, wobei das chirale Hilfsreagenz zurückgewonnen wird.

4. Verfahren zur Herstellung eines 1,2,3,4-Tetrahydroisochinolin-N-acyl-3,3-dicarbonsäuredialkylesters der Formel

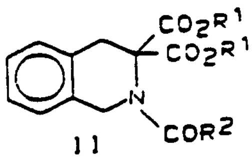

II

worin

$R^1$ $(C_1\text{-}C_4)$-Alkyl und
$R^2$ H, $(C_1\text{-}C_4)$-Alkyl oder $(C_6\text{-}C_{12})$-Aryl bedeuten,

dadurch gekennzeichnet, daß man

$a_1$) eine Verbindung der Formel IVa oder IVb

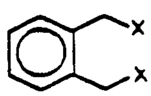

IVa: X=Cl

IVb: X=Br

mit N-Acylamidomalonsäuredialkylestern der Formel $(CO_2R^1)_2CHNHCOR^2$, worin $R^1$ und $R^2$ wie oben definiert sind, im basischen Milieu zu den obengenannten 1,2,3,4-Tetrahydroisochinolin-N-acyl-3,3-dicarbonsäuredialkylestern cyclisiert.

5. Verfahren gemäß Anspruch 4 zur Herstellung von 1,2,3,4-Tetrahydroisochinolin-N-acyl-3,3-dicarbonsäuredimethylester, dadurch gekennzeichnet, daß man eine Verbindung der Formel IVa oder IVb mit N-Acylamidomalonsäuredimethylestern der Formel $(CO_2CH_3)CHNHCOR^2$, worin $R^2$ die obengenannte Bedeutung hat, cyclisiert.

6. Verfahren zur Herstellung von 1,2,3,4-Tetrahydroisochinolin-3-carbonsäurebenzylestermandelat, dadurch gekennzeichnet, daß man

eine Verbindung der Formel I

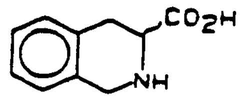

I

worin

$R^1$ $(C_1\text{-}C_4)$-Alkyl und
$R^2$ H, $(C_1\text{-}C_4)$-Alkyl oder $(C_6\text{-}C_{12})$-Aryl bedeuten,

mittels Benzylalkohol und p-Toluolsulfonsäure zur Verbindung der Formel VI verestert,

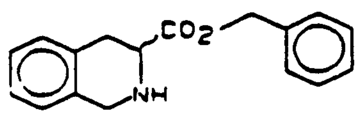

VI

die Verbindung der Formel VI mit D(-)Mandelsäure zu den Verbindungen der Formeln VIIa und VIIb bzw. mit L(+)Mandelsäure zu den Verbindungen der Formeln VIIIa und VIIIb umsetzt,

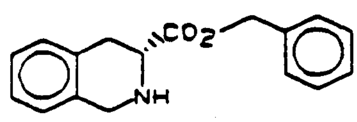

* D (-) Mandelsaure

VIIa

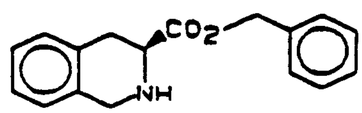

* D (-) Mandelsaure

VIIb

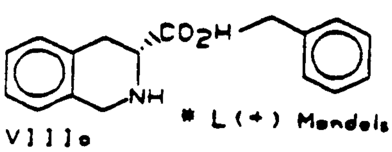

* L (+) Mandelsaure

VIIIa

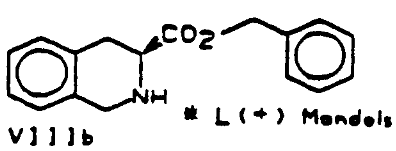

* L (+) Mandelsaure

VIIIb

anschließend die Verbindungen der Formeln VIIa und VIIb bzw. VIIIa und VIIIb durch fraktionierte Kristallisation in einem inerten Lösungsmittel in die optischen Antipoden trennt.

**7.** Verfahren gemäß Anspruch 6, dadurch gekennzeichnet daß (D)-1,2,3,4-Tetrahydroisochinolin-3-carbonsäurebenzylester-(D)-mandelat hergestellt wird.

**8.** Verfahren gemäß Anspruch 6, dadurch gekennzeichnet daß (L)-1,2,3,4-Tetrahydroisochinolin-3-carbonsäurebenzylester-(D)-mandelat hergestellt wird.

**9.** Verfahren gemäß Anspruch 6, dadurch gekennzeichnet daß (D)-1,2,3,4-Tetrahydroisochinolin-3-carbonsäurebenzylester-(L)-mandelat hergestellt wird.

**10.** Verfahren gemäß Anspruch 6, dadurch gekennzeichnet daß (L)-1,2,3,4-Tetrahydroisochinolin-3-carbonsäurebenzylester-(L)-mandelat hergestellt wird.

**11.** Verfahren zur Herstellung von (D,L)-1,2,3,4-Tetrahydroisochinolin-3-carbonsäure-(-)-menthylester, dadurch gekennzeichnet, daß man

$a_1$) eine Verbindung der Formel IVa oder IVb

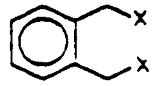

IVa: X=Cl

IVb: X=Br

mit N-Acylamidomalonsäuredialkylestern der Formel $(CO_2R^1)_2CHNHCOR^2$
worin

$R^1$ $(C_1-C_4)$-Alkyl und
$R^2$ H, $(C_1-C_4)$-Alkyl oder $(C_6-C_{12})$-Aryl bedeuten,

im basischen Milieu zu Verbindungen der Formel II

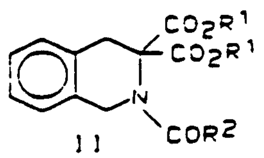

II

cyclisiert,
worin $R^1$ und $R^2$ wie oben definiert sind,
die so erhaltenen Verbindungen der Formel II durch basische Verseifung und nachfolgende saure Aufarbeitung zu Verbindungen der Formel III

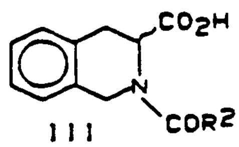

III

decarboxyliert und anschließend im sauren Milieu zu der Verbindungen der Formel I

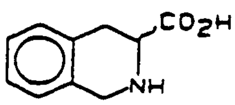

I

umsetzt, oder
$a_2$) die Verbindungen der Formeln IVa bzw. IVb im basischen Milieu zu den Verbindungen der Formel II cyclisiert und diese ohne Isolierung im Eintopfverfahren direkt zur Verbindungen der Formel I umsetzt,
und die racemischen Verbindungen der Formel I mit (-)Menthol und p-Toluolsulfonsäure zu einem Diastereomerenpaar der Formeln Va bzw. Vb

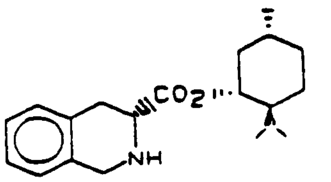

Va

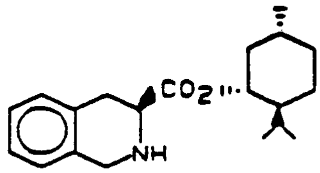

Vb

umsetzt.

## Claims

**Claims for the following Contracting States : AT, BE, CH, DE, DK, FR, GB, GR, IT, LI, LU, NL, PT, SE**

**1.** A process for the preparation of a compound of the formula I, Ia or Ib

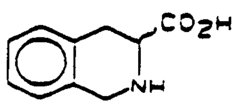

I

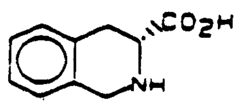

Ia

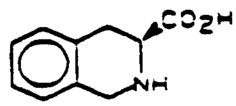

Ib

which comprises

$a_1$) cyclizing a compound of the formula IVa or IVb

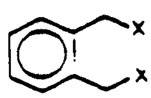

IVa: X = Cl

IVb: X = Br

with dialkyl N-acylamidomalonates of the formula $(CO_2R^1)_2CHNHCOR^2$ in which

$R^1$ is $(C_1\text{-}C_4)$-alkyl, and
$R^2$ is H, $(C_1\text{-}C_4)$-alkyl or $(C_6\text{-}C_{12})$-aryl,

in basic medium to give compounds of the formula II

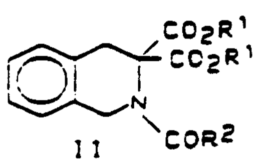

II

in which $R^1$ and $R^2$ are as defined above, decarboxylating the compounds of the formula II thus obtained by

basic hydrolysis and subsequent acid work-up to give compounds of the formula III

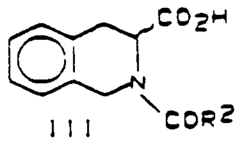

III

and then reacting in acid medium to give the compound of the formula I, or

$a_2$) cyclizing the compounds of the formula IVa or IVb in basic medium to give the compounds of the formula II and reacting these directly to give the compound of the formula I without isolation in a one-pot process,

if desired reacting the racemic compound of the formula I

$b_1$) with (-)menthol and p-toluenesulfonic acid to give a diastereomer pair of the formula Va or Vb

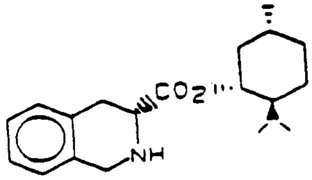

Va

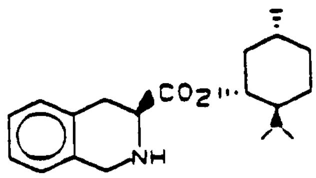

Vb

then separating the diastereomers by column chromatography and hydrolyzing by means of a base to give the compounds of the formula Ia or Ib or

$b_2$) esterifying the compound of the formula I by means of benzyl alcohol and p-toluenesulfonic acid to give the compound of the formula VI

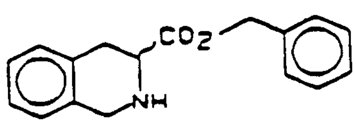

VI

reacting the compound of the formula VI with D(-)mandelic acid to give the compounds of the formulae VIIa and VIIb or with L(+)mandelic acid to give the compounds of the formulae VIIIa and VIIIb

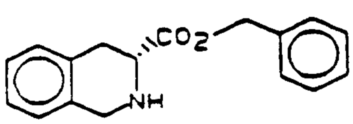

* D(-) mandelic acid

VIIa

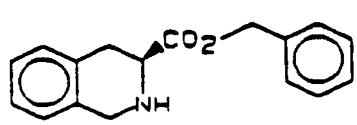

* D(-) mandelic acid

VIIb

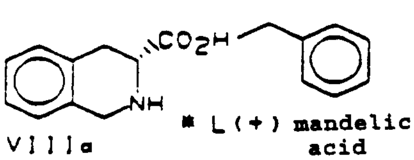

VIIIa * L(+) mandelic acid

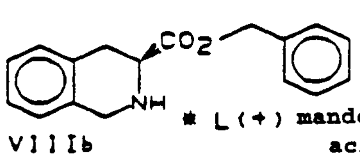

VIIIb * L(+) mandelic acid

then separating the compounds of the formulae VIIa and VIIb or VIIIa and VIIIb into the optical antipodes by

fractional crystallization in an inert solvent, and liberating the compounds of the formulae Ia and Ib by basic hydrolysis, the chiral auxiliary reagent being recovered.

2. The process for the preparation of a compound of the formula I, Ia or Ib as claimed in claim 1, wherein a compound of the formula IVa or IVb is reacted in a one-pot process to give the compound of the formula I and, if desired, the compound of the formula I thus obtained is esterified by means of benzyl alcohol and p-toluenesulfonic acid to give the compound of the formula VI, the compound of the formula VI is reacted with D(-)mandelic acid to give the compounds of the formulae VIIa and VIIb or with L(+)mandelic acid to give the compounds of the formulae VIIIa and VIIIb, then the compounds of the formulae VIIa and VIIb or VIIIa and VIIIb are separated into the optical antipodes by fractional crystallization in an inert solvent, and the compounds of the formulae Ia and Ib are liberated by basic hydrolysis, the chiral auxiliary reagent being recovered.

3. The process for the preparation of a compound of the formula Ia or Ib as claimed in claim 1, wherein the racemic compound of the formula I is reacted with (-)menthol and p-toluenesulfonic acid to give a diastereomer pair of the formulae Va and Vb, then the diastereomers are separated by column chromatography and hydrolyzed by means of a base to give the compounds of the formula Ia or Ib or the compound of the formula I is esterified by means of benzyl alcohol and p-toluenesulfonic acid to give the compound of the formula VI, the compound of the formula VI is reacted with D(-)mandelic acid to give the compounds of the formulae VIIa and VIIb or with L(+)mandelic acid to give the compounds of the formulae VIIIa and VIIIb, then the compounds of the formulae VIIa and VIIb or VIIIa and VIIIb are separated into the optical antipodes by fractional crystallization in an inert solvent, and the compounds of the formulae Ia and Ib are liberated by basic hydrolysis, the chiral auxiliary reagent being recovered.

4. A dialkyl 1,2,3,4-tetrahydroisoquinoline-N-acyl-3,3-dicarboxylate,
dimethyl 1,2,3,4-tetrahydroisoquinoline-N-acyl-3,3-dicarboxylate,
benzyl (D)-1,2,3,4-tetrahydroisoquinoline-3-carboxylate (D)-mandelate,
benzyl (L)-1,2,3,4-tetrahydroisoquinoline-3-carboxylate (D)-mandelate,
benzyl (D)-1,2,3,4-tetrahydroisoquinoline-3-carboxylate (L)-mandelate,
benzyl (L)-1,2,3,4-tetrahydroisoquinoline-3-carboxylate (L)-mandelate,
(-)-menthyl (D,L)-1,2,3,4-tetrahydroisoquinoline-3-carboxylate.

**Claims for the following Contracting State : ES**

1. A process for the preparation of a compound of the formula I, Ia or Ib

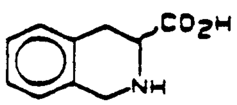
I

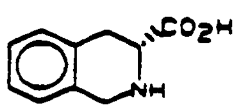
Ia

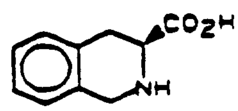
Ib

which comprises

a$_1$) cyclizing a compound of the formula IVa or IVb

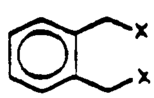

IVa: X=Cl

IVb: X=Br

with dialkyl N-acylamidomalonates of the formula $(CO_2R^1)_2CHNHCOR^2$ in which

$R^1$ is $(C_1$-$C_4)$-alkyl, and
$R^2$ is H, $(C_1$-$C_4)$-alkyl or $(C_6$-$C_{12})$-aryl,

in basic medium to give compounds of the formula II

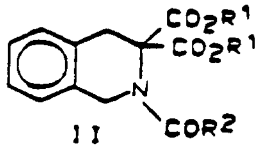

II

in which $R^1$ and $R^2$ are as defined above, decarboxylating the compounds of the formula II thus obtained by basic hydrolysis and subsequent acid work-up to give compounds of the formula III

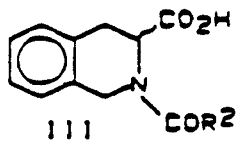

III

and then reacting in acid medium to give the compound of the formula I, or

$a_2$) cyclizing the compounds of the formula IVa or IVb in basic medium to give the compounds of the formula II and reacting these directly to give the compound of the formula I without isolation in a one-pot process,

if desired reacting the racemic compound of the formula I

$b_1$) with (-)menthol and p-toluenesulfonic acid to give a diastereomer pair of the formula Va or Vb

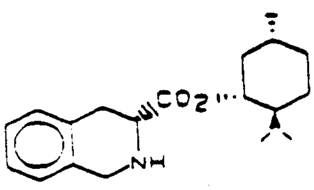

Va

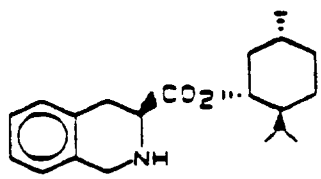

Vb

then separating the diastereomers by column chromatography and hydrolyzing by means of a base to give the compounds of the formula Ia or Ib or

$b_2$) esterifying the compound of the formula I by means of benzyl alcohol and p-toluenesulfonic acid to give the compound of the formula VI

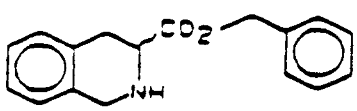

VI

reacting the compound of the formula VI with D(-)mandelic acid to give the compounds of the formulae VIIa

and VIIb or with L(+)mandelic acid to give the compounds of the formulae VIIIa and VIIIb

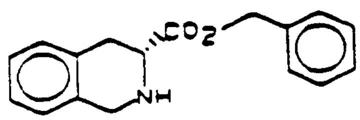

* D(-) mandelic acid

VIIa

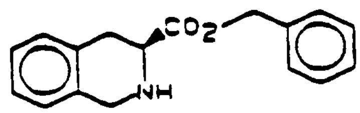

* D(-) mandelic acid

VIIb

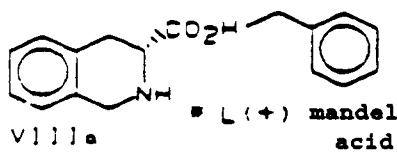

* L(+) mandelic acid

VIIIa

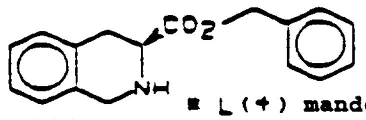

* L(+) mandelic acid

VIIIb

then separating the compounds of the formulae VIIa and VIIb or VIIIa and VIIIb into the optical antipodes by fractional crystallization in an inert solvent, and liberating the compounds of the formulae Ia and Ib by basic hydrolysis, the chiral auxiliary reagent being recovered.

**2.** The process for the preparation of a compound of the formula I, Ia or Ib as claimed in claim 1, wherein a compound of the formula IVa or IVb is reacted in a one-pot process to give the compound of the formula I and, if desired, the compound of the formula I thus obtained is esterified by means of benzyl alcohol and p-toluenesulfonic acid to give the compound of the formula VI, the compound of the formula VI is reacted with D(-)mandelic acid to give the compounds of the formulae VIIa and VIIb or with L(+)mandelic acid to give the compounds of the formulae VIIIa and VIIIb, then the compounds of the formulae VIIa and VIIb or VIIIa and VIIIb are separated into the optical antipodes by fractional crystallization in an inert solvent, and the compounds of the formulae Ia and Ib are liberated by basic hydrolysis, the chiral auxiliary reagent being recovered.

**3.** The process for the preparation of a compound of the formula Ia or Ib as claimed in claim 1, wherein the racemic compound of the formula I is reacted with (-)menthol and p-toluenesulfonic acid to give a diastereomer pair of the formulae Va and Vb, then the diastereomers are separated by column chromatography and hydrolyzed by means of a base to give the compounds of the formula Ia or Ib or the compound of the formula I is esterified by means of benzyl alcohol and p-toluenesulfonic acid to give the compound of the formula VI, the compound of the formula VI is reacted with D(-)mandelic acid to give the compounds of the formulae VIIa and VIIb or with L(+)mandelic acid to give the compounds of the formulae VIIIa and VIIIb, then the compounds of the formulae VIIa and VIIb or VIIIa and VIIIb are separated into the optical antipodes by fractional crystallization in an inert solvent, and the compounds of the formulae Ia and Ib are liberated by basic hydrolysis, the chiral auxiliary reagent being recovered.

**4.** A process for the preparation of a dialkyl 1,2,3,4-tetrahydroisoquinoline-N-acyl-3,3-dicarboxylate of the formula

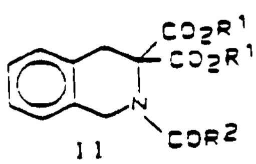

II

in which

$R^1$ is $(C_1\text{-}C_4)$-alkyl and
$R^2$ is H, $(C_1\text{-}C_4)$alkyl or $(C_6\text{-}C_{12})$-aryl,

which comprises

$a_1$) cyclizing a compound of the formula IVa or IVb

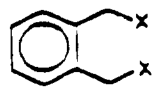

IVa: X=Cl

IVb: X=Br

with dialkyl N-acylamidomalonates of the formula $(CO_2R^1)_2CHNHCOR^2$, in which $R^1$ and $R^2$ are as defined above, in basic medium to give the abovementioned dialkyl 1,2,3,4-tetrahydroisoquinoline-N-acyl-3,3-dicarboxylates.

5. The process as claimed in claim 4 for the preparation of dimethyl 1,2,3,4-tetrahydroisoquinoline-N-acyl-3,3-dicarboxylate, wherein a compound of the formula IVa or IVb is cyclized with dimethyl N-acylamidomalonates of the formula $(CO_2CH_3)CHNHCOR^2$, in which $R^2$ has the abovementioned meaning.

6. A process for the preparation of benzyl 1,2,3,4-tetrahydroisoquinoline-3-carboxylate mandelate, which comprises esterifying a compound of the formula I

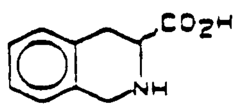

I

in which

$R^1$ is $(C_1-C_4)$-alkyl and
$R^2$ is H, $(C_1-C_4)$-alkyl or $(C_6-C_{12})$-aryl,

by means of benzyl alcohol and p-toluenesulfonic acid to give the compound of the formula VI

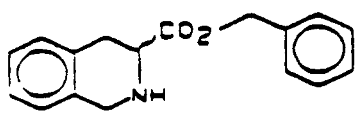

VI

reacting the compound of the formula VI with D(-)mandelic acid to give the compounds of the formulae VIIa and

VIIb or with L(+)mandelic acid to give the compounds of the formulae VIIIa and VIIIb

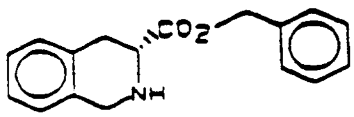

* D(-) mandelic acid

VIIa

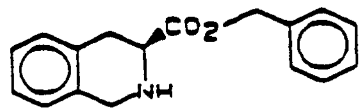

* D(-) mandelic acid

VIIb

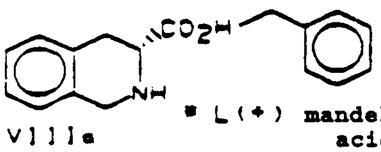

* L(+) mandelic acid

VIIIa

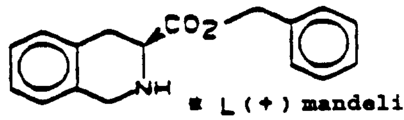

* L(+) mandelic acid

VIIIb

then separating the compounds of the formulae VIIa and VIIb or VIIIa and VIIIb into the optical antipodes by fractional crystallization in an inert solvent.

**7.** The process as claimed in claim 6, wherein benzyl (D)-1,2,3,4-tetrahydroisoquinoline-3-carboxylate (D)-mandelate is prepared.

**8.** The process as claimed in claim 6, wherein benzyl (L)-1,2,3,4-tetrahydroisoquinoline-3-carboxylate (D)-mandelate is prepared.

**9.** The process as claimed in claim 6, wherein benzyl (D)-1,2,3,4-tetrahydroisoquinoline-3-carboxylate (L)-mandelate is prepared.

**10.** The process as claimed in claim 6, wherein benzyl (L)-1,2,3,4-tetrahydroisoquinoline-3-carboxylate (L)-mandelate is prepared.

**11.** A process for the preparation of (-)-menthyl (D,L)-1,2,3,4-tetrahydroisoquinoline-3-carboxylate, which comprises

$a_1$) cyclizing a compound of the formula IVa or IVb

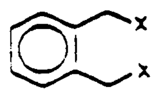

IVa: X=Cl

IVb: X=Br

with dialkyl N-acylamidomalonates of the formula $(CO_2R^1)_2CHNHCOR^2$ in which

$R^1$ is $(C_1$-$C_4)$-alkyl and

$R^2$ is H, $(C_1$-$C_4)$-alkyl or $(C_6$-$C_{12})$-aryl,

in basic medium to give compounds of the formula II

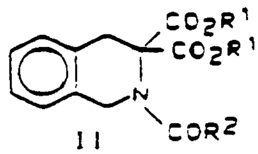

II

in which $R^1$ and $R^2$ are as defined above, decarboxylating the compounds of the formula II thus obtained by basic hydrolysis and subsequent acid work-up to give compounds of the formula III

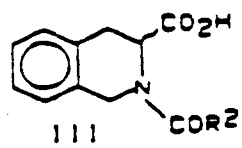

III

and then reacting in acidic medium to give the compound of the formula I

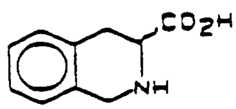

I

or

$a_2$) cyclizing the compounds of the formula IVa or IVb in basic medium to give the compounds of the formula II and reacting these directly to give the compound of the formula I without isolation in a one-pot process,

and reacting the racemic compound of the formula I with (-)menthol and p-toluenesulfonic acid to give a diastereomer pair of the formula Va or Vb

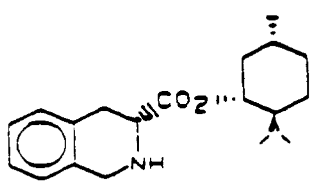

Va

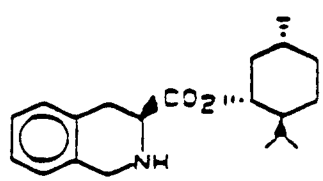

Vb

## Revendications

**Revendications pour les Etats contractants suivants : AT, BE, CH, DE, DK, FR, GB, GR, IT, LI, LU, NL, PT, SE**

**1.** Procédé de préparation d'un composé de formule I, Ia ou Ib

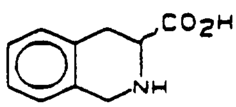

I

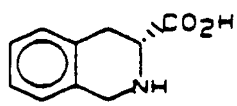

Ia

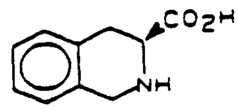

Ib

caractérisé en ce que

$a_1$) l'on cyclise en milieu basique un composé de formule IVa ou IVb

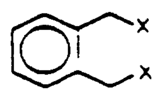

IVa: X=Cl

IVb: X=Br

avec des esters dialkyliques d'acide N-acylamidomalonique de formule $(CO_2R^1)_2CHNHCOR^2$ dans laquelle
$R^1$ représente un groupe alkyle en $C_1$-$C_4$ et
$R^2$ représente un atome d'hydrogène, un groupe alkyle en $C_1$-$C_4$ ou un groupe aryle en $C_6$-$C_{12}$,
pour former des composés de formule II

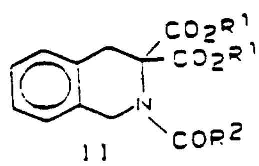

II

dans laquelle $R^1$ et $R^2$ sont définis comme précédemment, l'on décarboxyle les composés de formule II ainsi obtenus par saponification basique suivie d'un traitement acide pour donner un composé de formule III

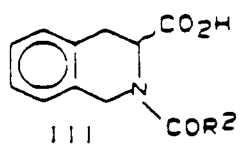

III

que l'on transforme en milieu acide en le composé de formule I, ou
$a_2$) l'on cyclise les composés de formule IVa ou IVb en milieu basique pour donner les composés de formule II que l'on transforme directement sans les isoler en composé de formule I dans un procédé en cuve unique, le cas échéant,

$b_1$) l'on fait réagir les composés racémiques de formule I avec du (-)menthol et de l'acide p-toluènesulfonique pour former un couple de diastéréoisomères de formule Va ou Vb,

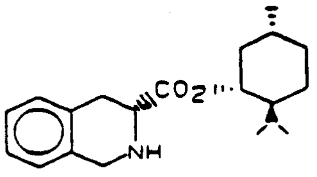

Va

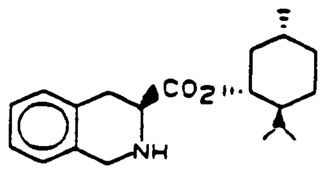

Vb

puis l'on sépare par chromatographie sur colonne les diastéréoisomères que l'on saponifie en milieu basique pour former les composés de formule Ia ou Ib, ou

$b_2$) l'on estérifie le composé de formule I au moyen d'alcool benzylique et d'acide p-toluènesulfonique pour former un composé de formule VI,

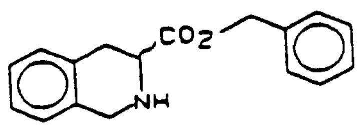

VI

l'on fait réagir le composé de formule VI avec de l'acide D(-)mandélique pour donner les composés de formules VIIa et VIIb, ou avec de l'acide L(+)mandélique pour donner les composés de formules VIIIa et VIIIb,

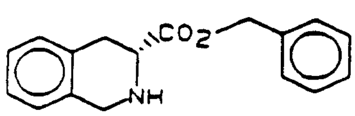

*Acide D(-)mandélique

VIIa

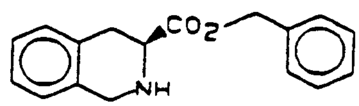

*Acide D(-)mandélique

VIIb

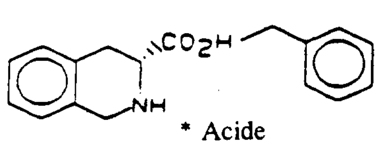

* Acide L(+)mandélique

VIIIa

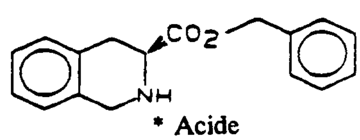

* Acide L(+)mandélique

VIIIb

puis l'on sépare les composés de formules VIIa et VIIb, ou VIIIa et VIIIb, en les isomères optiques par cristallisation fractionnée dans un solvant inerte, et l'on libère les composés de formules Ia et Ib par saponification basique, grâce à quoi l'on récupère l'auxiliaire réactionnel chiral.

2. Procédé de préparation d'un composé de formules I, Ia ou Ib, selon la revendication 1, caractérisé en ce que l'on fait réagir un composé de formule IVa ou IVb, dans un procédé en cuve unique pour former le composé de formule I et, le cas échéant, on estérifie le composé de formule I ainsi obtenu au moyen d'alcool benzylique et d'acide p-toluènesulfonique pour former un composé de formule VI, l'on fait réagir le composé de formule VI avec de l'acide D(-)mandélique pour former les composés de formules VIIa et VIIb, ou avec de l'acide L(+)mandélique pour former les composés de formules VIIIa et VIIIb, puis l'on sépare les composés de formules VIIa et VIIb, ou VIIIa et VIIIb, en les isomères optiques par cristallisation fractionnée dans un solvant inerte, et l'on libère les composés de formules Ia et Ib par saponification basique, grâce à quoi l'auxiliaire réactionnel chiral est récupéré.

3. Procédé de préparation d'un composé de formule Ia ou Ib, selon la revendication 1, caractérisé en ce que l'on fait réagir le composé racémique de formule I avec du (-)menthol et de l'acide p-toluènesulfonique pour former un couple de diastéréoisomères de formule Va ou Vb, puis l'on sépare par chromatographie sur colonne les diastéréoisomères que l'on saponifie en milieu basique pour former les composés de formule Ia ou Ib,
ou l'on estérifie le composé de formule I au moyen d'alcool benzylique et d'acide p-toluènesulfonique pour former le composé de formule VI, l'on fait réagir le composé de formule VI avec de l'acide D(-)mandélique pour former les composés de formules VIIa et VIIb, ou avec de l'acide L(+)mandélique pour former les composés de formules VIIIa et VIIIb, puis l'on sépare les composés de formules VIIa et VIIb, ou VIIIa et VIIIb, en les isomères optiques par cristallisation fractionnée dans un solvant inerte, et l'on libère les composés de formules Ia et Ib par saponification basique, grâce à quoi l'auxiliaire réactionnel chiral est récupéré.

4. Ester dialkylique d'acide 1,2,3,4-tétrahydroisoquinoléine-N-acyl-3,3-dicarboxylique,
ester diméthylique d'acide 1,2,3,4-tétrahydroisoquinoléine-N-acyl-3,3-dicarboxylique,
(D)-mandélate du (D)-1,2,3,4-tétrahydroisoquinoléine-3-carboxylate de benzyle,
(D)-mandélate du (L)-1,2,3,4-tétrahydroisoquinoléine-3-carboxylate de benzyle,
(L)-mandélate du (D)-1,2,3,4-tétrahydroisoquinoléine-3-carboxylate de benzyle,
(L)-mandélate du (L)-1,2,3,4-tétrahydroisoquinoléine-3-carboxylate de benzyle,
(D,L)- 1,2,3,4-tétrahydroisoquinoléine-3-carboxylate de (-)menthyle.

**Revendications pour l'Etat contractant suivant : ES**

1. Procédé de préparation d'un composé de formule I, Ia ou Ib

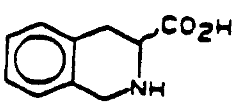
I

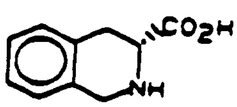
Ia

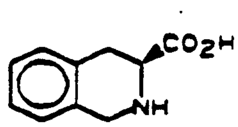
Ib

caractérisé en ce que

a$_1$) l'on cyclise en milieu basique un composé de formule IVa ou IVb

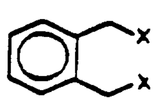
IVa : X=Cl
IVb : X=Br

avec des esters dialkyliques d'acide N-acylamidomalonique de formule $(CO_2R^1)_2CHNHCOR^2$ dans laquelle
$R^1$ représente un groupe alkyle en $C_1$-$C_4$ et
$R^2$ représente un atome d'hydrogène, un groupe alkyle en $C_1$-$C_4$ ou un groupe aryle en $C_6$-$C_{12}$,

pour former des composés de formule II

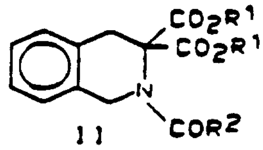

II

dans laquelle $R^1$ et $R^2$ sont définis comme précédemment, l'on décarboxyle les composés de formule II ainsi obtenus par saponification basique suivie d'un traitement acide pour donner un composé de formule III

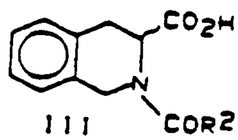

III

que l'on transforme en milieu acide en le composé de formule I, ou

$a_2$) l'on cyclise les composés de formule IVa ou IVb en milieu basique pour donner les composés de formule II que l'on transforme directement sans les isoler en composé de formule I dans un procédé en cuve unique, le cas échéant,

$b_1$) l'on fait réagir les composés racémiques de formule I avec du (-)menthol et de l'acide p-toluènesulfonique pour former un couple de diastéréoisomères de formule Va ou Vb,

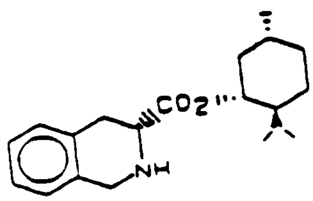

Va

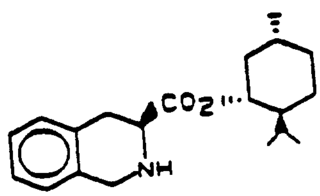

Vb

puis l'on sépare par chromatographie sur colonne les diastéréoisomères que l'on saponifie en milieu basique pour former les composés de formule Ia ou Ib, ou

$b_2$) l'on estérifie le composé de formule I au moyen d'alcool benzylique et d'acide p-toluènesulfonique pour former un composé de formule VI,

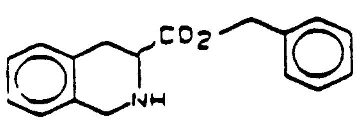

VI

l'on fait réagir le composé de formule VI avec de l'acide D(-)mandélique pour donner les composés de formules

VIIa et VIIb, ou avec de l'acide L(+)mandélique pour donner les composés de formules VIIIa et VIIIb,

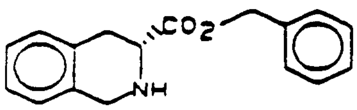

*Acide

D(–)mandélique

VIIa

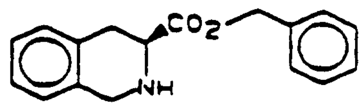

*Acide

D(–)mandélique

VIIb

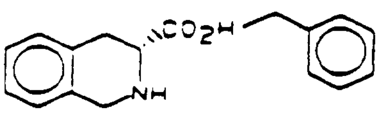

* Acide

L(+)mandélique

VIIIa

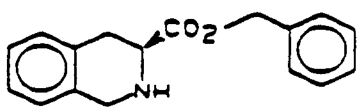

* Acide

L(+)mandélique

VIIIb

puis l'on sépare les composés de formules VIIa et VIIb, ou VIIIa et VIIIb, en les isomères optiques par cristallisation fractionnée dans un solvant inerte, et l'on libère les composés de formules Ia et Ib par saponification basique, grâce à quoi l'on récupère l'auxiliaire réactionnel chiral.

2. Procédé de préparation d'un composé de formule I, Ia ou Ib, selon la revendication 1, caractérisé en ce que l'on fait réagir un composé de formule IVa ou IVb dans un procédé en cuve unique pour former le composé de formule I et, le cas échéant, on estérifie le composé de formule I ainsi obtenu au moyen d'alcool benzylique et d'acide p-toluènesulfonique pour former un composé de formule VI, l'on fait réagir le composé de formule VI avec de l'acide D(-)mandélique pour former les composés de formules VIIa et VIIb, ou avec de l'acide L(+)mandélique pour former les composés de formules VIIIa et VIIIb, puis l'on sépare les composés de formules VIIa et VIIb, ou VIIIa et VIIIb, en les isomères optiques par cristallisation fractionnée dans un solvant inerte, et l'on libère les composés de formules Ia et Ib par saponification basique, grâce à quoi l'auxiliaire réactionnel chiral est récupéré.

3. Procédé de préparation d'un composé de formule Ia ou Ib, selon la revendication 1, caractérisé en ce que l'on fait réagir le composé racémique de formule I avec du (-)menthol et de l'acide p-toluènesulfonique pour former un couple de diastéréoisomères de formule Va ou Vb, puis l'on sépare par chromatographie sur colonne les diastéréoisomères que l'on saponifie en milieu basique pour former les composés de formule Ia ou Ib,
ou l'on estérifie le composé de formule I au moyen d'alcool benzilique et d'acide p-toluènesulfonique pour former le composé de formule VI, l'on fait réagir le composé de formule VI avec de l'acide (D)-mandélique pour former les composés de formules VIIa et VIIb, ou avec de l'acide L(+)mandélique pour former les composés de formules VIIIa et VIIIb, puis l'on sépare les composés de formules VIIa et VIIb, ou VIIIa et VIIIb, en les isomères optiques par cristallisation fractionnée dans un solvant inerte, et l'on libère les composés de formule Ia et Ib par saponification basique, grâce à quoi l'auxiliaire réactionnel chiral est récupéré.

4. Procédé de préparation d'un ester dialkylique d'acide 1,2,3,4-tétrahydroisoquinoléine-N-acyl-3,3-dicarboxylique de formule

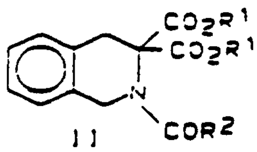

II

dans laquelle
$R^1$ représente un groupe alkyle en $C_1$-$C_4$ et
$R^2$ représente un atome d'hydrogène, un groupe alkyle en $C_1$-$C_4$ ou un groupe aryle en $C_6$-$C_{12}$,
caractérisé en ce que

$a_1$)l'on cyclise en milieu basique un composé de formule IVa ou IVb

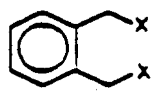

IVa: X=Cl

IVb: X=Br

avec des esters dialkyliques d'acide N-acylamidomalonique de formule $(CO_2R^1)_2CHNHCOR^2$, dans laquelle $R^1$ et $R^2$ sont définis comme précédemment,
pour former les esters dialkyliques d'acide 1,2,3,4-tétrahydroisoquinoléine-N-acyl-3,3-dicarboxylique mentionnés ci-dessus.

5. Procédé selon la revendication 4 de préparation d'ester diméthylique d'acide 1,2,3,4-tétrahydroisoquinoléine-N-acyl-3,3-dicarboxylique, caractérisé en ce que l'on cyclise un composé de formule IVa ou IVb avec des esters diméthyliques d'acide N-acylamidomalonique de formule $(CO_2CH_3)CHNHCOR^2$ dans laquelle $R^2$ a la signification précédente.

6. Procédé de préparation de mandélate 1,2,3,4-tétrahydroisoquinoléine-3-carboxylate de benzyle caractérisé en ce que l'on estérifie un composé de formule I

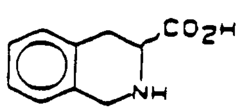

I

dans laquelle
$R^1$ représente un groupe alkyle en $C_1$-$C_4$ et
$R^2$ représente un atome d'hydrogène, un groupe alkyle en $C_1$-$C_4$ ou un groupe aryle en $C_6$-$C_{12}$,

au moyen d'alcool benzylique et d'acide p-toluènesulfonique pour former un composé de formule VI,

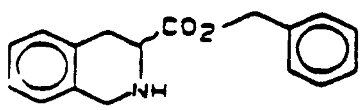

VI

l'on fait réagir le composé de formule VI avec de l'acide D(-)mandélique pour former les composés de formules VIIa et VIIb, ou avec de l'acide L(+)mandélique pour former les composés de formules VIIIa et VIIIb,

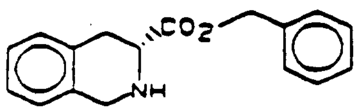

*Acide D(-)mandélique

VIIa

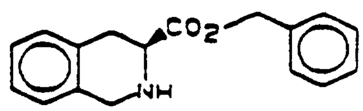

*Acide D(-)mandélique

VIIb

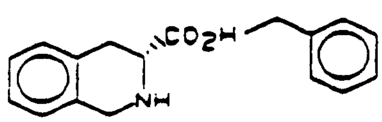

* Acide L(+)mandélique

VIIIa

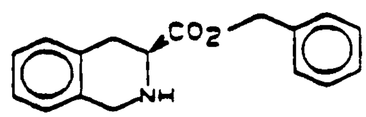

* Acide L(+)mandélique

VIIIb

puis l'on sépare les composés de formules VIIa et VIIb, ou VIIIa et VIIIb, en les isomères optiques par cristallisation fractionnée dans un solvant inerte.

7. Procédé selon la revendication 6, caractérisé en ce que l'on prépare le (D)-mandélate de (D)-1,2,3,4-tétrahydroisoquinoléine-3-carboxylate de benzyle.

8. Procédé selon la revendication 6, caractérisé en ce que l'on prépare le (D)-mandélate (L)-1,2,3,4-tétrahydroisoquinoléine-3-carboxylate de benzyle.

9. Procédé selon la revendication 6, caractérisé en ce que l'on prépare le (L)-mandélate de (D)-1,2,3,4-tétrahydroisoquinoléine-3-carboxylate de benzyle.

10. Procédé selon la revendication 6, caractérisé en ce que l'on prépare le (L)-mandélate de (L)-1,2,3,4-tétrahydroisoquinoléine-3-carboxylate de benzyle.

11. Procédé de préparation (D,L)-1,2,3,4-tétrahydroisoquinoléine-3-carboxylate de (-)menthyle, caractérisé en ce que

$a_1$) l'on cyclise en milieu basique un composé de formule IVa ou IVb

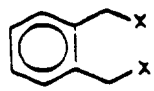

IVa : X = Cl

IVb : X = Br

avec des esters dialkyliques d'acide N-acylamidomalonique de formule $(CO_2R^1)_2CHNHCOR^2$ dans laquelle
$R^1$ représente un groupe alkyle en $C_1$-$C_4$ et
$R^2$ représente un atome d'hydrogène, un groupe alkyle en $C_1$-$C_4$ ou un groupe aryle en $C_6$-$C_{12}$,
pour former des composés de formule II

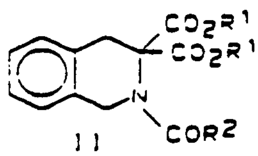

II

dans laquelle $R^1$ et $R^2$ sont définis comme précédemment, l'on décarboxyle les composés de formule II ainsi obtenus par saponification basique suivie d'un traitement acide pour donner un composé de formule III

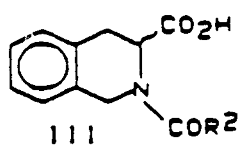

III

que l'on transforme en milieu acide en le composé de formule I,

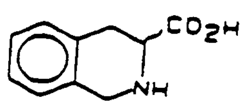

I

ou $a_2$) l'on cyclise les composés de formule IVa ou IVb en milieu basique pour former les composés de formule II que l'on transforme directement sans les isoler en composés de formule I dans un procédé en cuve unique, et l'on fait réagir les composés racémiques de formule I avec du (-)menthol et de l'acide p-toluènesulfonique

pour former un couple de diastéréoisomères de formule Va ou Vb.

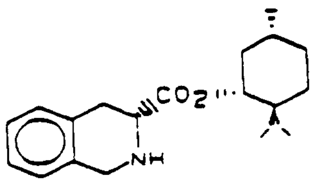

Va

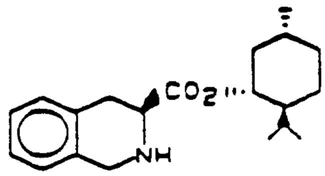

Vb